# EUROPÄISCHE PATENTSCHRIFT

(11) **EP 1 280 812 B1**
(45) Veröffentlichungstag und Bekanntmachung des Hinweises auf die Patenterteilung: **02.02.2005**
(21) Anmeldenummer: 01936275.5
(22) Anmeldetag: 25.04.2001
(51) Int. Cl.: C07F 9/6574, A61K 31/665, A61P 3/10, C12P 9/00, C12N 1/20

(54) **CYCLIPOSTINE, VERFAHREN ZU IHRER HERSTELLUNG UND VERWENDUNG DERSELBEN**
CYCLIPOSTINS, A METHOD FOR THEIR PRODUCTION AND THE USE OF THE SAME
CYCLIPOSTINES, LEUR PROCEDE DE PRODUCTION ET LEUR UTILISATION

(30) Priorität: 04.05.2000 DE 10021731
(43) Veröffentlichungstag der Anmeldung: 05.02.2003
(73) Patentinhaber: Aventis Pharma Deutschland GmbH, 65929 Frankfurt am Main (DE)
(72) Erfinder: VERTESY, Laszlo, 65817 Eppstein-Vockenhausen (DE); EHRLICH, Klaus, 65428 Rüsselsheim (DE); KURZ, Michael, 65719 Hofheim (DE); WINK, Joachim, 63322 Rödermark (DE)
(86) Internationale Anmeldenummer: PCT/EP2001/004652
(87) Internationale Veröffentlichungsnummer: WO 2001/083497

(56) Entgegenhaltungen:
- CHEMICAL ABSTRACTS, vol. 121, no. 7, 15. August 1994 (1994-08-15) Columbus, Ohio, US; abstract no. 81134, IZAWA, TAKEO ET AL: "Antibiotic NK901093A, its manufacture with Streptomyces, and insecticides and acaricides containing NK901093A" XP002175392 & JP 06 056859 A (NIPPON KAYAKU KK, JAPAN) 1. März 1994 (1994-03-01)
- CHEMICAL ABSTRACTS, vol. 118, no. 1, 4. Januar 1993 (1993-01-04) Columbus, Ohio, US; abstract no. 2472, KUROKAWA, TAKASHI ET AL: "Fermentative preparation of antibiotic NK901093 as insecticide and miticide." XP002175393 & JP 04 145089 A (NIPPON KAYAKU CO., LTD., JAPAN) 19. Mai 1992 (1992-05-19)
- TAKASHI KUROKAWA ET AL.: "Cyclophostin, acetylcholinesterase inhibitor from Streptomyces lavendulae" JOURNAL OF ANTIBIOTICS., Bd. 46, Nr. 8, August 1993 (1993-08), Seiten 1315-1318, XP002175390 JAPAN ANTIBIOTICS RESEARCH ASSOCIATION. TOKYO., JP ISSN: 0021-8820
- NEUMANN R. ET AL.: "Insecticidal organophosphates: nature made them first" EXPERIENTIA., Bd. 43, Nr. 11-12, 1. Dezember 1987 (1987-12-01), Seiten 1235-1237, XP002175391 BIRKHAUSER VERLAG. BASEL., CH ISSN: 0014-4754

## Beschreibung

Die Erfindung betrifft neue Verbindungen, genannt Cyclipostine, erhältlich durch Kulturvierung von *Streptomyces* species HAG 004107 (DSM 13381), und deren physiologisch verträglichen Salzen und chemischen Äquivalente. Die Erfindung betrifft weiterhin ein Verfahren zur Herstellung der Cyclipostine, den Mikroorganismus HAG 004107 (DSM 13381), die Verwendung der Cyclipostine und derer physiologisch verträglichen Salze und chemischen Äquivalente als Arzneimittel, insbesondere als Inhibitoren von Lipasen, sowie pharmazeutische Zubereitungen mit einem Gehalt von Cyclipostin oder ein physiologisch verträgliches Salz oder Äquivalent davon.

Eine Krankheit, die besonders vorteilhaft mit Lipase Inhibitoren behandelt werden kann, ist die Zuckerkrankheit Diabetes mellitus. Der Diabetes mellitus ist eine Erkrankung, die durch erhöhte Blutzucker-Konzentrationen aufgrund von chronischen Stoffwechselstörungen gekennzeichnet ist. Die Stoffwechselstörungen beruhen auf ein Mangel an Insulin oder herabgesetzter Insulin-Wirkung. Die fehlende Insulin-Wirkung führt zu mangelhafter Verwertung der ins Blut aufgenommenen Glucose durch die Körperzellen. Dadurch sowie durch Neubildung von Glucose aus Proteinen (Gluconeogenese) kommt es zum Anstieg des Blutglucosespiegels. Darüber hinaus führen bei verringerter Insulin-Wirkung im Fettgewebe die Insulin-antagonistischen Hormone, wie das Glucagon, zu gesteigerten Lipolyse und damit zu erhöhten Fettsäure-Konzentrationen im Blut. Es kommt zur Ketoacidose, d. h. zur gesteigerten Bildung von Keton-Körpern (Acetessigsäure, ß-Hydroxybuttersäure, Aceton). Unter akuten Bedingungen ist das Ausmaß der biochemischen Fehlregulierung lebensbedrohlich und führt, unbehandelt, zum diabetischem Koma, schließlich zum raschen Tod. Die Zuckerkrankheit gehört zu den häufigsten chronischen Stoffwechsel-Erkrankungen des Menschen, es wird geschätzt, daß bis über 3% der Bevölkerung eine diabetische oder prädiabetische Disposition aufweisen und damit akut bedroht sind.

Deshalb besteht ein großer Bedarf an Mitteln zur Behandlung oder Heilung des Diabetes mellitus.

Die Behandlung der Zuckerkrankheit erfolgt durch Insulingaben, beim Altersdiabetes, dem sogenannten nicht Insulinabhängigen (NIDDM) oder Typ II-Diabetes, werden zunächst Sulfonylhamstoffe verabreicht. Das Wirkprinzip der Sulfonylhamstoffe ist eine Vermehrung der Insulinausschüttung der ß-Zellen in der Bauchspeicheldüse um so den Hormonmangel oder die Insulin-Resistenz auszugleichen. Bei Fortschreiten der Erkrankung muß aber ebenfalls Insulin eingesetzt werden. Die Insulin-Wirkung kann man folgendermaßen zusammenfassen. Dieses Peptid-Hormon senkt die Konzentration der Glucose im Blut und führt zu einer Steigerung anaboler und gleichzeitig zu einer Hemmung kataboler Prozesse:
- Es steigert den Glucose-Transport in die Körperzellen,
- Es steigert die Glykogenbildung in der Leber und in den Muskeln,
- Es hemmt die Lipolyse,
- Es steigert die Aufnahme von Fettsäuren in das Fettgewebe und
- Es steigert die Aufnahme von Aminosäuren in die Körperzellen sowie die Protein-Synthese.

Einer der stärksten Effekte von Insulin ist die Hemmung der Lipolyse. Bei Typ II Diabetikern greift diese Regulierung der Lipolyse nicht mehr und es kommt zu einem erhöhten Spiegel an freien Fettsäuren im Blut. Freie Fettsäuren im Blut stimulieren die Gluconeogenese in der Leber und vermindern die Nutzung von Glucose in den Skelettmuskeln. Kontrolliert wird die Lipolyse, das heißt die Freisetzung von Fettsäuren durch die sogenannte Hormonsensitive Lipase (HSL), die sich in den Fettzellen befindet und von Insulin über eine Phosphorylierungs-Kaskade gehemmt wird. Es wären deshalb Inhibitoren, das heißt Hemmstoffe der HSL wünschenswert, die die Insulin-Wirkung simulieren und den Blutfettspiegel zu senken vermögen. Solche Agenzien sind geeignet zur Behandlung von Typ II-Diabetikern zur Kontrolle des Fettstoffwechsels, aber auch bei anderen Speicherstoff-Erkrankungen wären Anwendungen möglich. Aus all diesen Gründen werden neue Inhibitoren der HSL und anderer Lipasen dringend benötigt und deshalb gesucht.

Es ist überraschend gefunden worden, daß der Mikroorganismus-Stamm *Streptomyces* species HAG 004107, DSM 13381, hoch wirksame neue Lipase-Inhibitoren zu bilden vermag, die die hormonsensitive Lipase noch in sehr geringen Konzentrationen hemmen. Die neuen natürlichen Verbindungen sind Organo-Phosphate, die aus einem zweifachen Ringsystem (Bicyclus) und einer substituierten Kohlenstoffkette bestehen und die Lipasen spezifisch hemmen. Das Ringgerüst ist erstmals - nur mit einer Methylgruppe anstelle der Kohlenstoffkette - als ein Acetylcholin-Estrase - Inhibitor, CGA 134 736, von R. Neumann & H. H. Peter in Experientia, Band 43, Seite 1235-1237, 1987 und später die gleiche Verbindung, als Cyclophostin bezeichnet, von T. Kurokawa et al, im J. Antibiotics, 46, 1315-1318, 1993 beschrieben worden. Diese strukturverwandte Verbindung weist keine selektive Lipase-hemmende Eigenschaft auf. Die bisher bekannten Substanzen weisen Nachteile auf, die sich in unbefriedigender Wirkhöhe, hoher Toxizität und/oder unerwünschten Nebenwirkungen äußern.

Die vorliegende Erfindung betrifft daher Verbindungen der allgemeinen Formel I 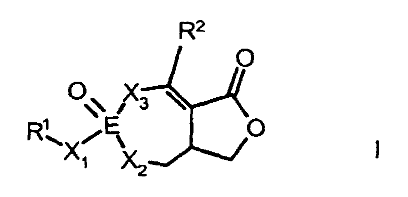 worin
R¹ für
1. eine Kohlenstoff-Kette mit 10 bis 18 C - Atomen steht, die geradkettig, verzweigt, gesättigt oder ungesättigt, carbo- oder heterocyclisch sein kann und worin die Kohlenstoff-Kette gegebenenfalls ein- oder zweifach substituiert ist durch:
   1.1 -OH,
   1.2 =O,
   1.3 -O-C₁-C₆-Alkyl, worin Alkyl gerade oder verzweigt ist,
   1.4 -O-C₂-C₆-Alkenyl, worin Alkenyl gerade oder verzweigt ist,
   1.5 -C₁-C₆-Alkyl, worin Alkyl gerade oder verzweigt ist,
   1.6 - Aryl,
   1.7 -C₁-C₆-Alkylbenzol,
   1.8 - Diphenyl,
   1.9 -NH-C₁-C₆-Alkyl, worin Alkyl gerade oder verzweigt ist,
   1.10 -NH-C₂-C₆-Alkenyl, worin Alkenyl gerade oder verzweigt ist,
   1.11 -NH₂,
   1.12 =S,
   1.13 -S-C₁-C₆-Alkyl, worin Alkyl gerade oder verzweigt ist, oder
   1.14 -S-C₂-C₆-Alkenyl, worin Alkenyl gerade oder verzweigt ist,
   1.15 Halogen, worin die Substituenten 1.1 bis 1.15 auch noch substituiert sein können;
R² für
1. C₁-C₆-Alkyl, worin Alkyl unsubstituiert oder ein- oder zweifach wie unter 1.1 bis 1.15 beschrieben substituiert ist ,
2. C₂-C₆-Alkenyl, worin Alkenyl unsubstituiert oder ein- oder zweifach wie unter 1.1 bis 1.15 beschrieben substituiert ist, oder
3. C₂-C₆-Alkinyl, worin Alkinyl unsubstituiert oder ein- oder zweifach wie unter 1.1 bis 1.15 beschrieben substituiert ist, steht,
E für Phosphor- (P) und
X₁, X₂ und X₃ unabhängig voneinander für
1. -O-.
in allen ihren stereochemischen Formen und Gemische dieser Formen in jedem Verhältnis, sowie deren physiologisch verträglichen Salzen.

R¹ hat eine Kettenlänge von 10 bis 18 C-Atomen. Die Kette kann gesättigt sein, z.B. -Alkyl, worin Alkyl gerade oder verzweigt vorliegen kann, oder ungesättigt, z.B. -Alkenyl oder -Alkinyl, worin Alkenyl oder Alkinyl gerade oder verzweigt ist. R₁ kann unsubstituiert oder ein- oder zweifach, gleich oder verschieden substituiert sein mit Gruppen 1.1 bis 1.15, wie oben beschrieben. Bevorzugt sind die Substituenten an den Kohlenstoffatomen 8' bis 16', besonders bevorzugt sind die Positionen 10' bis 14'. Die Substituenten 1.1 bis 1.15 können auch noch substituiert sein durch eine oder mehrere Gruppen ausgewählt aus: Alkohol-, Aldehyd-, Acetal-, Ketal-, Ether-, Carboxyl-, Ester-, Amino-, Nitril-, Nitro-, Oxim-, Oximether und Halogen.

Eine carbocyclische Kohlenstoff-Kette mit 10 bis 18 C-Atomen bedeutet eine aus 10 bis 18-C Atomen bestehende Kette mit einer oder mehreren, vorzugsweise mit einem, mit zwei oder mit drei Ringsystemen, die vorzugsweise jeweils aus 4, 5, 6 oder 7-Kohlenstoffatomen bestehen. Die Ringe können mono-, di- oder tricyclisch, vorzugsweise monocyclisch, vorliegen und am Anfang, in der Mitte und/oder am Ende der Kohlenstoff-Kette positioniert sein. Die Carbocyclen können aliphatisch oder von aromatischer Natur sein. Beispiele sind: substituierte Diphenyle oder Alkylbenzole.

Eine heterocyclische Kohlenstoff-Kette mit 10 bis 18 C-Atomen bedeutet eine aus 10 bis 18-C Atomen bestehende Kette mit einer oder mehreren, vorzugsweise mit ein bis drei Ringsystemen, bei denen mindestens ein KohlenstoffAtom durch Hetero-Atome, wie z.B. O, S oder N ersetzt ist. Die Ringe können mono-, di- oder tricyclisch, vorzugsweise monocyclisch, vorliegen und am Anfang, in der Mitte und/oder am Ende der Kohlenstoff-Kette positioniert sein. Es können vorzugsweise 4-, 5-, 6- oder 7-gliedrige Ringe sein, aliphatisch oder von aromatischer Natur. Beispiele sind: Alkyl-Piperidine, substituiert oder unsubstituiert.

Halogen bedeutet Chlorid, Bromid, Fluorid oder Pseudohalogenide, wie Cyanid (Nitril).

-C₁-C₆-Alkyl bedeutet ein geradkettiges oder verzweigtes Alkyl mit 1, 2, 3, 4, 5 oder 6 C-Atomen, wie z.B. Methyl, Ethyl, i-Propyl, tert.Butyl und Hexyl.

-C₂-C₆-Alkenyl bedeutet ein geradkettiges oder verzweigtes Alkenyl mit 2, 3, 4, 5 oder 6 C-Atomen, wie z.B. Allyl, Crotyl und Pentenyl.

-C₂-C₆-Alkinyl bedeutet ein geradkettiges oder verzweigtes Alkinyl mit 2, 3, 4, 5 oder 6 C-Atomen, wie z.B. Propinyl, Butinyl und Pentinyl.

R¹ steht vorzugsweise für
1. -(CH₂)₁₅CH₃.
2. -(CH₂)₁₃CH(CH₃)₂,
3. -(CH₂)₁₁CH(OH)(CH₂)₃CH₃,
4. -(CH₂)₁₁CH(OH)CH₂CH(CH₃)₂,
5. -(CH₂)₁₂CH(OH)(CH₂)₂CH₃,
6. -(CH₂)₁₃CH(OH)CH₂CH₃,
7. -(CH₂)₁₄CH(OH)CH₃,
8. -(CH₂)₁₅CH₂(OH),
9. -(CH₂)₁₆CH₃, oder
10.0 -(CH₂)₁₃C=OCH₂CH₃
11.0 -(CH₂)₁₂C=OCH₂CH₂CH₃
12.0 -(CH₂)₁₁C=OCH₂CH₂CH₂CH₃
13.0 -(CH₂)₁₃CH₃
14.0 -(CH₂)₁₁CH(CH₃)₂
15.0 -(CH₂)₁₄CH₃ oder
16.0 -(CH₂)₁₂CH(CH₃)₂

R² steht vorzugsweise für C₁-C₆-Alkyl, insbesondere Methyl, Ethyl oder Propyl.

Bevorzugte Verbindungen der Erfindung sind unten angegeben:
Cyclipostin A der Formel II: 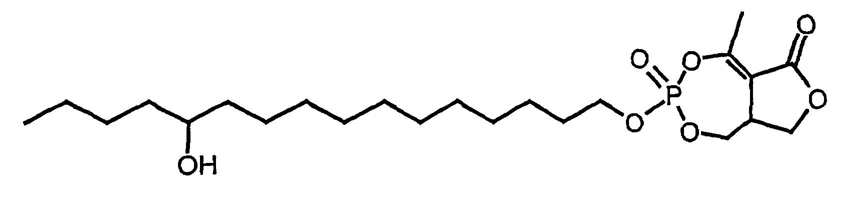
Cyclipostin A 2 der Formel II A. 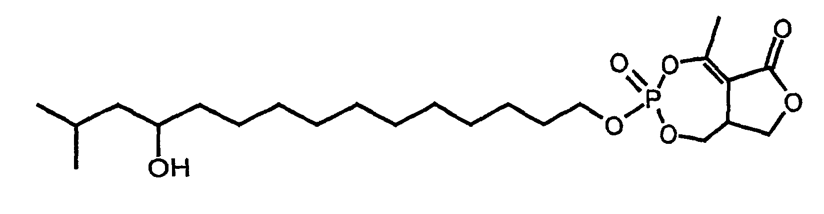
Cyclipostin B der Formel III: 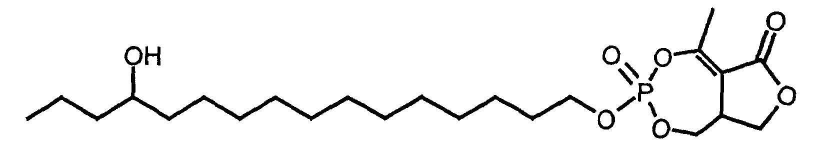
Cyclipostin C der Formel IV: 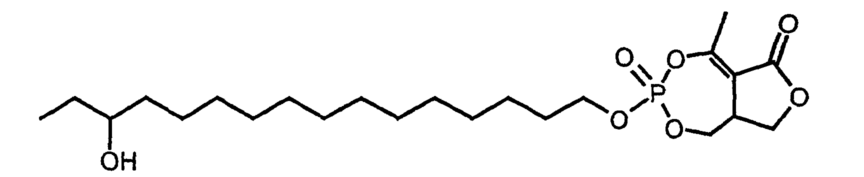
Cyclipostin D der Formel V: 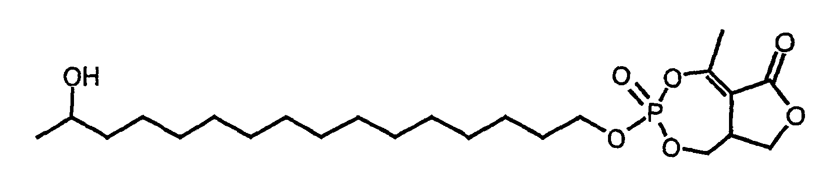
Cyclipostin E der Formel VI: 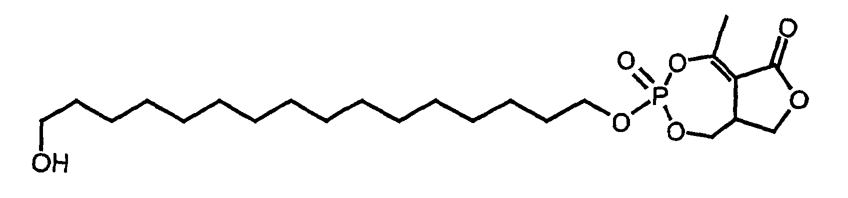
Cyclipostin F der Formel VII: 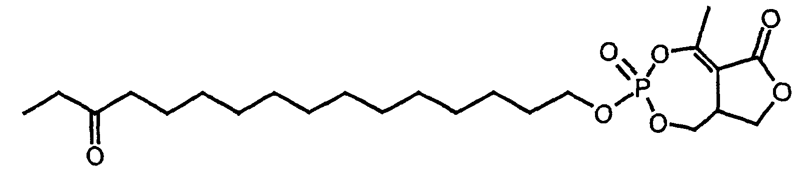
Cyclipostin G der Formel VIII: 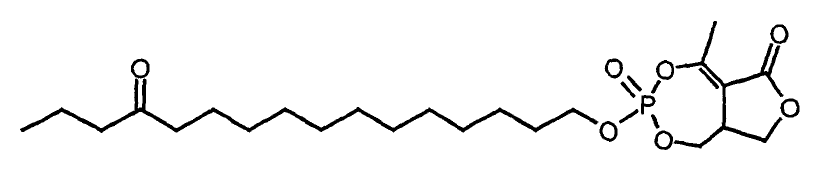
Cyclipostin H der Formel IX: 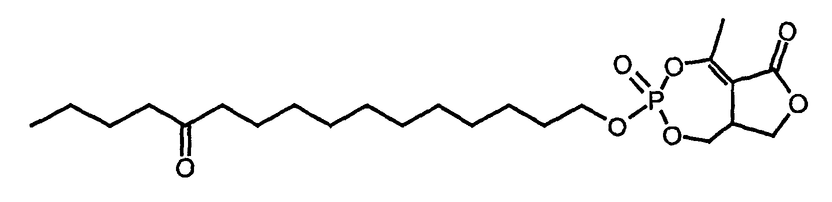
Cyclipostin N der Formel X: 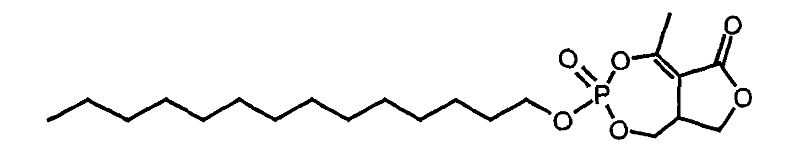
Cyclipostin P der Formel XI: 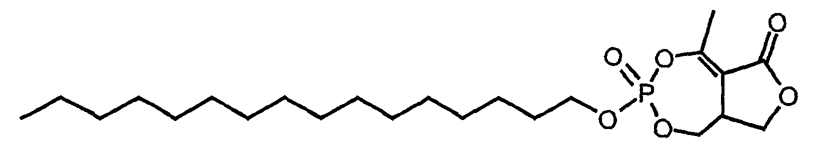
Cyclipostin P 2 der Formel XI A: 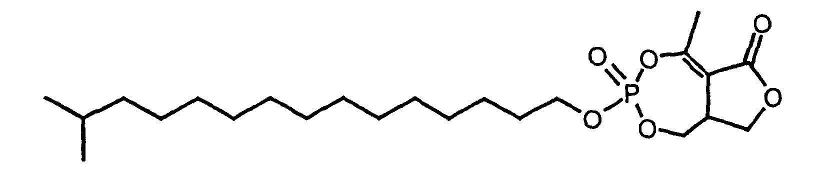
Cyclipostin Q der Formel XII: 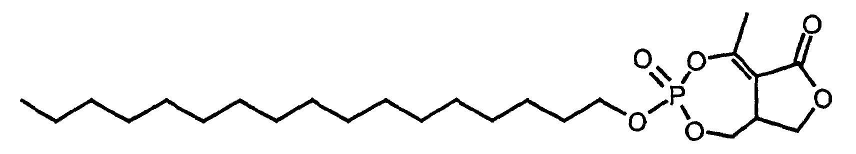
Cyclipostin R der Formel XIII: 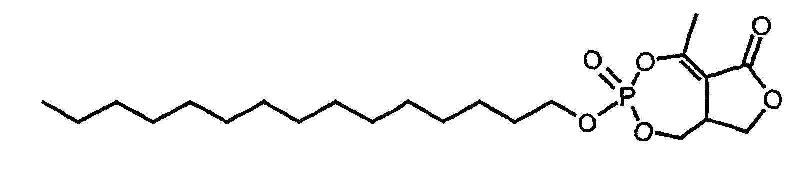
Cyclipostin R2 der Formel XIIIA: 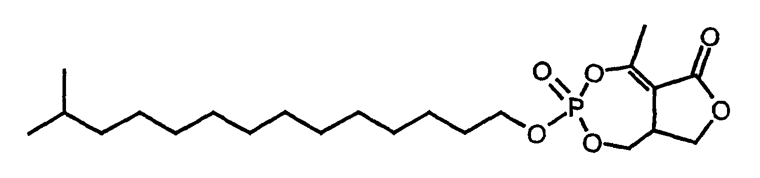
Cyclipostin S der Formel XIV: 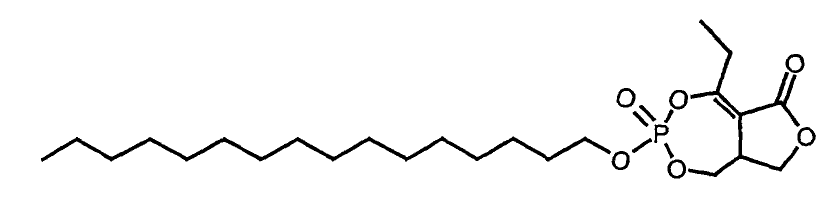
Cyclipostin T der Formel XV: 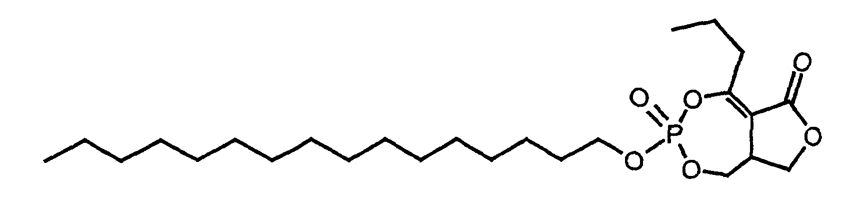
Cyclipostin T2 der Formel XV A: 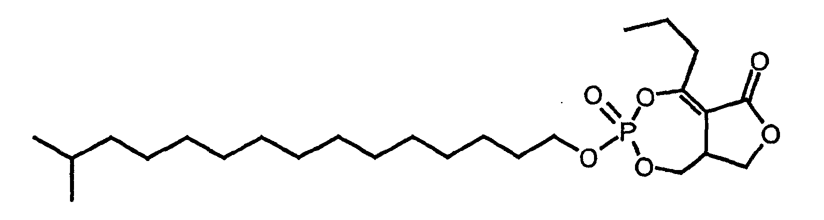 allen ihren stereochemischen Formen und Gemische dieser Formen in jedem Verhältnis, sowie deren physiologisch verträgliche Salze.

Die Zählweise der Kohlenstoff-Atome für die NMR-Spektren in den oben genannten Formeln lautet:

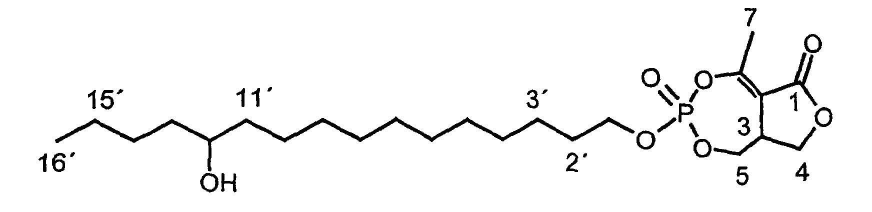

Allein das Ringsystem enthält zwei asymetrisch substituierte Atome, die C-Atome 3 und das Phosphor-Atom. Beide Atome können in der R- oder in der S-Konfiguration vorliegen. Es ist überraschend gefunden worden, daß der Stamm *Streptomyces* species HAG 004107, DSM 13381, in der Lage ist, jeweils mehrere Stereoisomere der Verbindungen der allgemeinen Formel I zu bilden, das heißt der Stamm Verbindungen synthetisiert, bei denen die Atome C3 und P unabhängig voneinander die R- oder die S-Konfiguration einnehmen können. Isomere mit der räumlichen Form am Kohlenstoff(3) in R- und am Phosphor in S- Konfiguration kommen in Kulturen des *Streptomyces* species HAG 004107, DSM 13381, vermehrt vor. Formel I A:

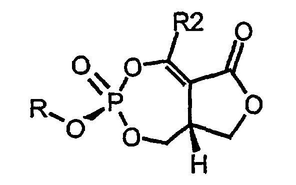

Daneben werden aber auch Cyclipostine mit anderen Konfigurationen, wie (R,R), (S,S) oder (S,R) gebildet, die überraschender Weise ebenfalls beträchtliche Lipase-hemmende Wirkungen aufweisen.

Die Verbindung der Formel I oder ein physiologisch verträgliches Salz oder chemische Äquivalent davon ist herstellbar indem der Mikroorganismus *Streptomyces* species HAG 004107, DSM 13381, oder einer seiner Varianten oder Mutanten unter geeigneten Bedingungen in einem Kulturmedium fermentiert wird, bis sich eine oder mehrere Verbindungen der allgemeinen Formel I in dem Kulturmedium anhäufen und anschließend aus dem Kulturmedium isoliert und gegebenenfalls in chemische Äquivalente und physiologisch verträgliche Salze überführt werden.

Die erfindungsgemäßen Cyclipostine können durch *Actinomycetales* species bevorzugt durch *Streptomyces* species HAG 004107, DSM 13381 produziert werden. *Streptomyces* species HAG 004107, DSM 13381 besitzt ein elfenbeinfarbenes Mycel (RAL 1014) und ist durch die für Streptomyceten charakteristischen Conidiophoren gekennzeichnet.

Ein Isolat wurde bei der Deutschen Sammlung von Mikroorganismen und Zellkulturen GmbH, Mascheroder Weg 1B, D 38124 Braunschweig, Deutschland, nach den Regeln des Budapester Vertrages am 16. März 2000 unter der folgenden Nummer hinterlegt: *Streptomyces* species HAG 004107, DSM 13381.

Anstelle des Stammes *Streptomyces* species HAG 004107, DSM 13381 können auch dessen Mutanten und Varianten eingesetzt werden, die eine oder mehrere Verbindungen der erfindungsgemäßen Cyclipostine synthetisieren. Solche Mutanten können in an sich bekannter Weise durch physikalische Mittel, beispielsweise Bestrahlung, wie mit Ultraviolett- oder Röntgenstrahlen, oder chemische Mutagene, wie beispielsweise Ethylmethansulfonat (EMS), 2-Hydroxy-4-methoxy-benzophenon (MOB) oder N-Methyl-N'-nitro-N-nitrosoguanidin (MNNG) erzeugt werden.

Die Erfindung betrifft somit ein Verfahren zur Herstellung einer Verbindung der Formel I oder ein physiologisch verträgliches Salz davon, dadurch gekennzeichnet, daß der Mikroorganismus *Streptomyces* species HAG 004107, DSM 13381, oder einer seiner Varianten oder Mutanten unter geeigneten Bedingungen in einem Kulturmedium fermentiert wird, bis sich eine oder mehrere Verbindungen der allgemeinen Formel I in dem Kulturmedium anhäufen und anschließend aus dem Kulturmedium isoliert und gegebenenfalls in chemische Äquivalente und physiologisch verträgliche Salze überführt werden.

Vorzugsweise wird der Stamm *Streptomyces* species HAG 004107, DSM 13381, seine Mutanten und/oder Varianten in einer Nährlösung (auch als Kulturmedium bezeichnet) mit Kohlenstoff und Stickstoffquelle sowie den üblichen anorganischen Salzen fermentiert, bis sich die neuen Cyclipostine in dem Kulturmedium anhäufen, anschließend werden die Cyclipostine aus dem Kulturmedium isoliert und gegebenenfalls in die einzelnen aktiven Komponenten aufgetrennt.

Die Fermentation wird vorzugsweise unter aeroben Bedingungen durchgeführt, sie verläuft besonders gut bei einer Temperatur zwischen 18 und 35°C und bei einem pH zwischen 6 und 8.

Das erfindungsgemäße Verfahren kann für die Fermentation im Labormaßstab (Milliliter- bis Literbereich) und für den industriellen Maßstab (Kubikmetermaßstab) eingesetzt werden. Alle Prozentangaben beziehen sich, wenn nichts anderes angegeben ist, auf das Gewicht. Mischungsverhältnisse bei Flüssigkeiten beziehen sich auf das Volumen, wenn keine anderen Angaben gemacht werden.

Als bevorzugte Kohlenstoffquellen für die aerobe Fermentation eignen sich assimilierbare Kohlenhydrate und Zuckeralkohole, wie Glukose, Laktose, Saccharose oder D-Mannit sowie kohlenhydrathaltige Naturprodukte, wie z.B. Haferflocken, Sojamehl und Malzextrakt. Als stickstoffhaltige Nährstoffe kommen in Betracht: Aminosäuren, Peptide und Proteine sowie deren Abbauprodukte, wie Peptone oder Tryptone, ferner Fleischextrakte, Hefeextrakte, gemahlene Samen, beispielsweise von Mais, Weizen, Bohnen, Soja oder der Baumwollpflanze, Destillationsrückstände der Alkoholherstellung, Fleischmehle oder Hefeextrakte, aber auch Ammoniumsalze und Nitrate. An anorganischen Salzen kann die Nährlösung beispielsweise Chloride, Carbonate, Sulfate oder Phosphate der Alkalioder Erdalkalimetalle, Eisen, Zink, Kobalt und Mangan enthalten.

Die Bildung der erfindungsgemäßen Cyclipostine der Formel II bis XV A verläuft besonders gut in einem Kulturmedium, das etwa 0,1 bis 5 %, bevorzugt 0,3 bis 3 % Haferflocken und Spurenelemente enthalten. Die Angaben in Prozent sind jeweils bezogen auf das Gewicht des gesamten Kulturmediums.

Die bevorzugte Bildung der Cyclipostine der Formel VIII bis XV A kann besonders gut durchgeführt werden in Nährlösungen, die etwa 0,1 bis 5 %, bevorzugt 0,3 bis 2 % Glycerin und 0,2 bis 5 %, bevorzugt 0,5 bis 3 % Sojamehl und 0,05 bis 1,0 g/l, bevorzugt 0,1 bis 1,0 g/l Natriumchlorid enthalten.

In dem Kulturmedium bildet *Streptomyces* species HAG 004107, DSM 13381, ein Gemisch aus Cyclipostinen. Je nach Zusammensetzung des Kulturmediums kann der mengenmäßige Anteil eines oder mehrerer der erfindungsgemäßen Cyclipostine variieren. Außerdem kann durch die Medienzusammensetzung die Synthese einzelner Cyclipostine gesteuert werden, so daß eines oder auch mehrere der Cyclipostine gar nicht bzw. in einer Menge unterhalb der Nachweisgrenze vom Mikroorganismus hergestellt werden.

Vorzugsweise enthält die Kultur ein nachweisbares Cyclipostin. Bevorzugt werden die Cyclipostine A oder P oder P 2 gebildet.

Neben den Cyclipostinen A bis T2 (Verbindungen der Formel II bis XV A) werden in dem Kulturmedium des *Streptomyces* species HAG 004107, DSM 13381, weitere verwandte Verbindungen gebildet, die sich durch veränderte Reste R¹ und R² von den in den Formeln II bis XV A wiedergegebenen Verbindungen unterscheiden. In kleineren Mengen wurden Cyclipostine nachgewiesen, die einen verkürzten oder weiter verzweigten Rest R¹ aufwiesen. Auch Oxidations- (Hydroxylierungs-)produkte dieser Nebenkomponenten sind in Kulturen des *Streptomyces* HAG 004107, DSM 13381, nachweisbar.

Die Kultivierung des Mikroorganismus erfolgt aerob, also beispielsweise submers unter Schütteln oder Rühren in Schüttelkolben oder Fermentem, gegebenenfalls unter Einführen von Luft oder Sauerstoff. Sie kann in einem Temperaturbereich von etwa 18 bis 35°C, vorzugsweise bei etwa 25 bis 32°C, insbesondere bei 26 bis 30°C durchgeführt werden. Der pH-Bereich sollte zwischen 6 und 8 liegen, vorzugsweise zwischen 6,5 und 7,8. Man kultiviert den Mikroorganismus unter diesen Bedingungen im allgemeinen über einen Zeitraum von 24 bis 300 Stunden, vorzugsweise 30 bis 90 Stunden.

Vorteilhaft kultiviert man in mehreren Stufen, d. h. man stellt zunächst eine oder mehrere Vorkulturen in einem flüssigen Kulturmedium her, die dann in das eigentliche Produktionsmedium, die Hauptkultur, beispielsweise im Volumenverhältnis 1:10, überimpft werden. Die Vorkultur erhält man, z.B., indem man ein Mycel in eine Nährlösung überimpft und etwa 36 bis 120 Stunden, bevorzugt 48 bis 96 Stunden, wachsen läßt. Das Mycel kann beispielsweise erhalten werden, indem man den Stamm etwa 3 bis 40 Tage, bevorzugt 4 bis 10 Tage, auf einem festen oder flüssigen Nährboden, beispielsweise Malz-Hefe-Agar oder Haferflocken-Agar wachsen läßt.

Der Fermentationsverlauf kann anhand des pH-Wertes der Kulturen oder des Mycelvolumens sowie durch chromatographische Methoden, wie z.B. Dünnschichtchromatographie oder High Pressure Liquid Chromatography oder Ausprüfen der biologischen Aktivität überwacht werden. Die erfindungsgemäßen Cyclipostine sind im Mycel, zum keineren Teil auch im Kulturfiltrat enthalten. Das im folgenden beschriebene IsolierungsverFahren dient zur Aufreinigung der erfindungsgemäßen Cyclipostine, vorzugsweise zur Aufreinigung der Cyclipostine A und P.

Die Isolierung bzw. Aufreinigung der erfindungsgemäßen Cyclipostine aus dem Kulturmedium erfolgt nach bekannten Methoden unter Berücksichtigung der chemischen, physikalischen und biologischen Eigenschaften der Naturstoffe. Zum Testen der Cyclipostin-Konzentration im Kulturmedium oder in den einzelnen Isolierungsstufen kann die Dünnschichtchromatographie, beispielsweise an Kieselgel mit Methylenchlorid-Ethylacetat oder Chloroform-Methanol -- Gemischen (z. B. im Mengenverhältnis 98:1) als Laufmittel oder HPLC verwendet werden. Die Detektion bei der dünnschichtchromatographischen Auftrennung kann beispielsweise durch Färbereagentien wie Molybdatophosphorsäure oder J₂-Dampf erfolgen, wobei die Menge der gebildeten Substanz zweckmäßig mit einer Eichlösung verglichen wird.

Zur Isolierung der erfindungsgemäßen Cyclipostine wird das Mycel zunächst von dem Kulturmedium nach den üblichen Verfahren abgetrennt und anschließend werden die Cyclipostine von der Zellmasse mit einem gegebenenfalls mit Wasser mischbaren organischen Lösungsmittel extrahiert. Die organische Lösungsmittelphase enthält die erfindungsgemäßen Cyclipostine, sie werden gegebenenfalls im Vakuum konzentriert und wie unten beschrieben weiter aufgereinigt.

Das Kulturfiltrat wird gegebenenfalls mit dem Konzentrat des Mycel-Extraktes vereinigt und mit einem geeigneten, mit Wasser nicht mischbarem organischen Lösungsmittel, zum Beispiel mit n-Butanol oder Ethylacetat, extrahiert. Die anschließend abgetrennte organische Phase wird ggf. im Vakuum konzentriert und in 1/30 des ursprünglichen Volumens Wasser/Methanol gelöst.

Die weitere Aufreinigung eines oder mehrerer der erfindungsgemäßen Cyclipostine erfolgt durch Chromatographie an geeigneten Materialien, vorzugsweise z.B. an Molekularsieben, an Normal-Phasenträger, wie z. B. Kieselgel, Aluminiumoxid, an Ionenaustauschern oder an Adsorberharzen bzw. an Umkehr-Phasen (reversed phase, RP). Mit Hilfe dieser Chromatographie-Verfahren werden die Cyclipostine getrennt. Die Chromatographie der Cyclipostine erfolgt mit organischen Lösungsmitteln oder mit Gemischen von wässrigen und organischen Lösungen.

Unter Gemischen von wässrigen oder organischen Lösungen versteht man alle mit Wasser mischbaren organischen Lösemittel, vorzugsweise Methanol, Propanol und Acetonitril, in einer Konzentration von 10 bis 100 % Lösemittel, vorzugsweise 60 bis 90 % Lösemittel oder auch alle gepufferten wässrigen Lösungen, die mit organischen Lösemitteln mischbar sind. Die zu verwendeten Puffer sind die gleichen wie oben angegeben.

Die Trennung der Cyclipostine aufgrund ihrer unterschiedlichen Polarität erfolgt mit Hilfe der Reversed Phase Chromatographie, beispielsweise an MCl® (Adsorberharz von Mitsubishi, Japan) oder Amberlite XAD® (TOSOHAAS), an weiteren hydrophoben Materialien, wie zum Beispiel an RP-8- oder RP-18-Phasen. Außerdem kann die Trennung mit Hilfe der Normalphasen-Chromatographie, zum Beispiel an Kieselgel, Aluminiumoxyd und ähnlichen erfolgen.

Die Chromatographie der Cyclipostine erfolgt mit gepufferten oder angesäuerten wäßrigen Lösungen oder Gemischen von wäßrigen Lösungen mit Alkoholen oder anderen, mit Wasser mischbaren organischen Lösemitteln. Als organisches Lösemittel wird vorzugsweise Propanol und Acetonitril verwendet.

Unter gepufferten oder angesäuerten wäßrigen Lösungen versteht man z.B. Wasser, Phosphatpuffer, Ammoniumacetat, Citratpuffer in einer Konzentration von 1 mM bis 0,5 M sowie Ameisensäure, Essigsäure, Trifluoressigsäure oder allen handelsüblichen, dem Fachmann bekannten Säuren, vorzugsweise in einer Konzentration von 0,01 bis 3 %, insbesondere 0,1 %.

Chromatographiert wird mit einem Gradienten, der mit 100 % wäßrigem Puffer beginnt und mit 100 % Lösemittel endet, vorzugsweise wird ein linearer Gradient von 50 bis 100 % 2-Propanol oder Acetonitril gefahren.

Alternativ kann auch eine Gelchromatographie oder die Chromatographie an hydrophoben Phasen erfolgen.

Die Gelchromatographie wird an Polyacrylamid- oder Mischpolymergelen durchgeführt, wie z.B. Biogel-P 2® (Fa. Biorad), Fractogel TSK HW 40® (Fa. Merck, Deutschland oder Toso Haas, USA) oder an Sephadex® (Fa. Pharmacia, Uppsala, Schweden).

Die Reihenfolge der vorgenannten Chromatographien ist umkehrbar.

Ein weiterer, sehr wirksamer Reinigungsschritt für Cyclipostine ist die Kristallisation. Die Cyclipostine kristallisieren aus Lösungen in organischen Lösungsmitteln und aus Mischungen von Wasser mit organischen Lösungsmitteln. Die Kristallisation wird in an sich bekannter Weise, zum Beispiel durch Konzentrieren oder Abkühlen gesättigter Cyclipostin-Lösungen, durchgeführt.

Die erfindungsgemäßen Cyclipostine sind im festen oder flüssigen Zustand und in Lösungen im pH-Bereich zwischen 4 und 8, insbesondere 5 und 7, stabil und lassen sich damit in übliche galenische Zubereitungen einarbeiten.

Ester, und Ether Derivate, Oxidations- Hydrierungs- sowie Reduktionsprodukte können nach in der Literatur beschriebenen Verfahren, z.B., in "Advanced Organic Synthesis, 4^{th} Edition, J. March, John Wiley & sons., 1992, hergestellt werden.

Die vorliegende Erfindung umfaßt allen stereoisomeren Formen der Verbindungen der Formel I bis XV A. In den Verbindungen der Formel I bis XV A enthaltene Asymmetriezentren können alle unabhängig voneinander die S-Konfiguration oder die R-Konfiguration aufweisen. Zur Erfindung gehören alle möglichen Enantiomeren und Diastereomeren, ebenso Mischungen von zwei oder mehr stereoisomeren Formen, zum Beispiel Mischungen von Enantiomeren und/oder Diastereomeren, in allen Verhältnissen. Enantiomere sind also in enantiomerenreiner Form, sowohl als linksdrehende als auch als rechtsdrehende Antipoden, R- und S-Konfigurationen, in Form von Racematen und in Form von Mischungen der beiden Enantiomeren in allen Verhältnissen Gegenstand der Erfindung. Bei Vorliegen einer cis/trans-Isomerie sind sowohl die cis-Form als auch die trans-Form und Gemische dieser Formen in allen Verhältnissen Gegenstand der Erfindung.

Aufgrund ihrer wertvollen pharmakologischen Eigenschaften eignen sich die erfindungsgemäßen Verbindungen zur Anwendung als Arzneimittel in der Humanund/oder Tiermedizin. Sie hemmen Lipasen und besitzen zur Behandlung von Stoffwechselerkrankungen, die ihre Ursache in der Störung des Lipidstoffwechsels haben, günstige Eigenschaften. Die erfindungsgemäßen Verbindungen der allgemeinen Formel I besitzen eine überraschende hemmende Wirkung an der hormonsensitiven Lipase, HSL, einem allosterischen Enzym in Adipozyten, das durch Insulin gehemmt wird und für den Abbau von Fetten in Fettzellen und damit für die Überführung von Fettbestandteilen in die Blutbahn verantwortlich ist. Eine Hemmung dieses Enzyms entspricht also einer insulinähnlichen Wirkung der erfindungsgemäßen Verbindungen, die letztlich zu einer Verminderung von freien Fettsäuren im Blut und von Blutzucker führt. Sie können also eingesetzt werden bei Entgleisungen des Stoffwechsels wie zum Beispiel beim nicht insulinabhängigen Diabetes mellitus, beim diabetischen Syndrom und bei direkter Schädigung des Pankreas.

Die Erfindung betrifft somit pharmazeutische Zubereitungen, die eine oder mehrere der erfindungsgemäßen Cyclipostine und/oder chemische Äquivalente davon enthalten. Bevorzugt ist die Verwendung in Mischung mit geeigneten Hilfsstoffen oder Trägermaterial. Als Trägermaterial können beim Menschen alle pharmakologisch verträglichen Trägermaterialien und/oder Hilfsstoffe verwendet werden.

Die Erfindung betrifft weiterhin ein Verfahren zur Herstellung eines erfindungsgemäßen Arzneimittels, das dadurch gekennzeichnet ist, daß man mindestens eine der erfindungsgemäßen Verbindungen mit einem pharmazeutisch geeigneten und physiologisch verträglichen Träger und gegebenenfalls weiteren geeigneten Wirk-, Zusatz- oder Hilfsstoffen in eine geeignete Darreichungsform bringt.

Die erfindungsgemäßen Arzneimittel werden im allgemeinen oral, lokal oder parenteral verabreicht, aber auch eine rektale Anwendung ist prinzipiell möglich. Geeignete feste oder flüssige galenische Zubereitungsformen sind beispielsweise Granulate, Pulver, Tabletten, Dragees, (Mikro-)Kapseln, Zäpfchen, Sirupe, Emulsionen, Suspensionen, Aerosole, Tropfen oder injizierbare Lösungen in Ampullenform sowie Präparate mit protrahierter Wirkstoff-Freigabe, bei deren Herstellung üblicherweise Trägerstoffe und Zusätze und/oder Hilfsmittel wie Spreng-, Binde-, Überzugs-, Quellungs-, Gleit- oder Schmiermittel, Geschmacksstoffe, Süßungsmittel oder Lösungsvermittler Verwendung finden. Als häufig verwendete Träger- oder Hilfsstoffe seien z.B. Magnesiumcarbonat, Titandioxid, Laktose, Mannit und andere Zucker, Talkum, Micheiweiß, Gelatine, Stärke, Vitamine, Cellulose und ihre Derivate, tierische oder pflanzliche Öle, Polyethylenglykole und Lösungsmittel, wie etwa steriles Wasser, Alkohole, Glycerin und mehrwertige Alkohole genannt.

Gegebenenfalls können die Dosierungseinheiten für die orale Verabreichung mikroverkapselt werden, um die Abgabe zu verzögern oder über einen längeren Zeitraum auszudehnen, wie beispielsweise durch Überziehen oder Einbetten des Wirkstoffs in Teilchenform in geeignete Polymere, Wachse oder dergleichen.

Vorzugsweise werden die pharmazeutischen Präparate in Dosierungseinheiten hergestellt und verabreicht, wobei jede Einheit als aktiven Bestandteil eine bestimmte Dosis einer oder mehrerer Verbindungen der erfindungsgemäßen Cyclipostine und/oder chemische Derivate davon enthält. Bei festen Dosierungseinheiten wie Tabletten, Kapseln und Suppositorien kann diese Dosis bis zu etwa 200 mg, bevorzugt jedoch etwa 0,1 bis 100 mg, und bei Injektionslösungen in Ampullenform bis zu etwa 200 mg, vorzugsweise aber etwa 0,1 bis 100 mg, pro Tag betragen.

Die zu verabreichende Tagesdosis ist abhängig vom Körpergewicht, Alter, Geschlecht und Zustand des Säugers. Unter Umständen können jedoch auch höhere oder niedrigere Tagesdosen angebracht sein. Die Verabreichung der Tagesdosis kann sowohl durch Einmalgabe in Form einer einzelnen Dosierungseinheit oder aber in mehreren kleineren Dosierungseinheiten als auch durch Mehrfachgabe unterteilter Dosen in bestimmten Intervallen erfolgen. Die Erfindung betrifft auch pharmazeutische Zubereitungen, die eine oder mehrere der erfindungsgemäßen Cyclipostine und/oder chemische Derivate davon enthalten. Bevorzugt ist die Verwendung in Mischung mit geeigneten Hilfsstoffen oder Trägermaterial. Als Trägermaterial können beim Menschen alle pharmakologisch verträglichen Trägermaterialien und/oder Hilfsstoffe verwendet werden.

Die Wirkung der erfindungsgemäßen Verbindungen der Formel I wurde an folgendem Enzymtestsytem geprüft:

### Enzympräparation:

### Präparation der partiell gereinigten HSL:

Isolierte Rattenfettzellen werden aus Nebenhodenfettgewebe von nicht-behandelten männlichen Ratten (Wistar, 220-250 g) durch Kollagenasebehandlung gemäß publizierter Verfahren gewonnen (z.B. S. Nilsson et al., Anal. Biochem. 158, 1986, 399 - 407; G. Fredrikson et al., J. Biol. Chem. 256,1981, 6311 - 6320; H. Tomquist et al., J. Biol. Chem. 251, 1976, 813 - 819). Die Fettzellen aus 10 Ratten werden dreimal durch Flotation mit jeweils 50 ml Homogenisationspuffer (25 ml Tris/HCl, pH 7.4, 0.25 M Sucrose, 1 mM EDTA, 1 mM DTT, 10 µg/ml Leupeptin, 10 µg/ml Antipain, 20 µg/ml Pepstatin) gewaschen und schließlich in 10 ml Homogenisationspuffer aufgenommen. Die Fettzellen werden im Teflon-in-Glas Homogenisator (Braun-Melsungen) durch 10 Hübe bei 1500 rpm und 15°C homogenisiert. Das Homogenisat wird zentrifugiert (Sorvall SM24-Röhrchen, 5000 rpm, 10 min, 4°C). Der Unterstand zwischen der oben liegenden Fettschicht und dem Pellet wird abgenommen und die Zentrifugation wiederholt. Der daraus resultierende Unterstand wird erneut zentrifugiert (Sorvall SM24-Röhrchen, 20000 rpm, 45 min, 4°C). Der Unterstand wird abgenommen und mit 1 g Heparin-Sepharose (Pharmacia-Biotech, CL-6B, 5 x gewaschen mit 25 mM Tris/HCl, pH 7.4, 150 mM NaCl) versetzt. Nach Inkubation für 60 imin bei 4°C (in Intervallen von 15 min aufschütteln) wird der Ansatz zentrifugiert (Sorvall SM24-Röhrchen, 3000 rpm, 10 min, 4°C). Der Überstand wird durch Zugabe von Eisessig auf pH 5.2 gebracht und 30 min bei 4°C inkubiert. Die Präzipitate werden durch Zentrifugation (Sorvall SS34, 12000 rpm, 10 min, 4°C) gesammelt und in 2.5 ml 20 mM Tris/HCl, pH 7.0, 1 mM EDTA, 65 mM NaCl, 13 % sucrose, 1 mM DTT, 10 µg/ml Leupeptin/Pepstatin/Antipain suspendiert. Die Suspension wird über Nacht bei 4°C gegen 25 mM Tris/HCl, pH 7.4, 50 % Glyzerin, 1 mM DTT, 10 µg/ml Leupeptin, Pepstätin, Antipain dialysiert und dann auf eine Hydroxiapatit-Säule aufgetragen (0.1 g pro 1 ml Suspension, äquilibriert mit 10 mM Kaliumphosphat, pH 7.0, 30 % Glyzerin, 1 mM DTT). Die Säule wird mit vier Volumina Äquilibrierungspuffer bei einer Flußrate von 20 bis 30 ml/h gewaschen. Die HSL wird mit einem Volumen Äquilibrierungspuffer, der 0.5 M Kaliumphosphat enthält, eluiert, sodann dialysiert (s.o.) und 5- bis 10-fach konzentriert durch Ultrafiltration (Amicon Diaflo PM 10 Filter) bei 4°C. Die partiell gereinigte HSL kann 4 bis 6 Wochen bei -70°C aufbewahrt werden.

### Assay:

Für die Herstellung des Substrats werden 25-50 µCi [³H]Trioleoylglycerin (in Toluol), 6.8 µMol unmarkiertes Trioleoylglycerin und 0.6 mg Phospholipide (Phosphatidylcholin/Phosphatidylinositol 3:1 w/v) gemischt, über N₂ getrocknet und darin in 2 ml 0.1 M KPᵢ (pH 7.0) durch Ultraschallbehandlung (Branson 250, Mikrospitze, Einstellung 1-2, 2 x 1 min im 1-min Intervall) aufgenommen. Nach Zugabe von 1 ml KPᵢ und erneuter Ultraschallbehandlung (4 x 30 sec auf Eis in 30-sec Intervallen) wird 1 ml 20% BSA (Bovine serum albumin) (in KPᵢ) hinzugefügt (Endkonzentration Trioleoylglyzerin 1.7 mM). Für die Reaktion werden 100 µl Substratlösung zu 100 µl HSL-Lösung (HSL präpariert wie oben, verdünnt in 20 mM KPᵢ, pH 7.0, 1 mM EDTA, 1 mM DTT, 0.02% BSA, 20 µg/ml Pepstatin, 10 µg/ml Leupeptin) pipettiert und für 30 min bei 37°C inkubiert. Nach Zugabe von 3.25 ml Methanol/Chloroform/Heptan (10:9:7) und von 1.05 ml 0.1 M K₂CO₃, 0.1 M Borsäure (pH 10.5) wird gut gemischt und schließlich zentrifugiert (800 x g, 20 min). Nach Phasentrennung wird ein Äquivalent der oberen Phase (1 ml) abgenommen und die Radioaktivität durch Flüssigszintillationsmessung bestimmt.

### Auswertung:

Substanzen werden üblicherweise in vier unabhängigen Ansätzen geprüft. Die Hemmung der enzymatischen Aktivität der HSL durch eine Testsubstanz wird durch den Vergleich mit einer nicht-gehemmten Kontrollreaktion bestimmt. Die Berechnung des IC₅₀-Wertes erfolgt über eine Hemmkurve mit mind. 10 Konzentrationen der Testsubstanz. Für die Analyse der Daten wird das Softwarepaket GRAPHIT, Elsevier-BIOSOFT , benutzt.
In diesem Test zeigten die Verbindungen die folgende Wirkung:
Die Cyclipostine A, P, P2 und R hemmen die Lipolyse in Ratten-Adipozyten mit IC₅₀ = ~ 0.2 µM, sie inhibieren die humane hormon-sensitive Lipase (HSL) mit
Trioleylglycerol als Substrat: IC₅₀ = ~ 0.07 bis 0.5 µM. Die HSL aus Ratten wird, mit NBD (4-Chloro-7-nitrobenzo-2-oxa-1,3-diazol) als Substrat, in Konzentrationen von 4 nM bis 10 nM gehemmt.

Die Cyclipostine hemmen sowohl die hormonsensitive Lipase (HSL), als auch die Mono-Acyl-Glycerin - Lipase des Rattenextraktes in submikromolaren Konzentrationen.

In den sich anschließenden Beispielen wird die Erfindung weiter erläutert. Prozentangaben beziehen sich auf das Gewicht. Mischungsverhältnisse bei Flüssigkeiten beziehen sich auf das Volumen, wenn keine anderen Angaben gemacht wurden.

### Beispiele

### Beispiel 1

### Herstellung einer Glycerinkultur von Streptomyces species HAG 004107, DSM 13381.

100 ml Nährlösung (Malzextrakt 2,0%, Hefeextract 0,2 %, Glucose 1,0 % (NH₄)₂HPO₄ 0,05 %, pH 6,0) in einem sterilen 300 ml Erlenmeyerkolben werden mit dem Stamm *Streptomyces* species HAG 004107, DSM 13381, beimpft und 7 Tage bei 28° C und 180 UpM auf einer rotierenden Schüttelmaschine inkubiert. 1,5 ml dieser Kultur werden anschließend mit 1,5 ml 99 %igem Glycerin verdünnt und bei -20° C gelagert.

### Beispiel 2

### Herstellung einer Vorkultur im Erlenmeyerkolben von Streptomyces species HAG 004107, DSM 13381,

Ein steriler 300 ml Erlenmeyerkolben mit 100 ml der folgende Nährlösung: 15 g/l Glucose, 15 g/l Sojamehl , 5 g/l Cornsteep, 2 g/l CaCO₃ und 5 g/l NaCl wird mit einer auf einem Schrägröhrchen (gleiche Nährlösung, jedoch mit 2 % Agar) gewachsenen Kultur oder mit 1 ml einer Glycerinkultur (s. Beispiel 1) beimpft und auf einer Schüttelmaschine bei 180 UpM und 28 °C inkubiert. Zum Animpfen von 10 und 200 I Fermentem genügt eine 48 bis 96 Std. alte Submerskultur (Animpfmenge ca. 10 %) aus der gleichen Nährlösung.

### Beispiel 3

### Herstellung einer Kultur im Erlenmeyerkolben von Streptomyces species HAG 004107, DSM 13381.

Sterile 300 ml Erlenmeyerkolben mit 100 ml der folgenden Nährlösung:
20 g/l Hundehaferflocken
2.5 ml Spurenelement-Lösung
werden mit 10% Animpfmenge aus der Vorkultur (Beispiel 2) beimpft und auf einer Schüttelmaschine bei 180 UpM und 28° C inkubiert. Die Kultur kann nach zwei Tagen zur Gewinnung von der Cyclipostine oder zum Animpfen von Fermentem verwendet werden. Die Spurenelement-Lösung hat folgende Zusammensetzung: 3 g/l CaCl₂ x 2H₂0
1 g/l Fe(III)-Citrat;
0.2 g/l MnSO₄ x H₂0;
0.1 g/l ZnCl₂;
0.025 g/l CuSO₄ x 5H₂O,
0.02 g/l Na-Tetraborat
0.004 g/l CoCl₂ x 6H₂O
0.01 g/l Na-Molybdat.

### Beispiel 4

### Herstellung der Cyclipostine der Formeln II bis IX.

Ein 200 I Fermenter wird mit 90 Liter Nährlösung unter folgenden Bedingungen betrieben:
- Nährmedium:: 20 g/l Haferflocken in Wasser;
2.5 ml/l Spurenelement.
pH 7.8 (vor der Sterilisation)

Die Nährlösung wird 30 Minuten lang hitzesterilisiert und nach dem Abkühlen 5% des Volumens mit Impfmaterial, gewonnen nach Beispiel 3 beimpft.
- Spurenelement-Lösung:: 3 g/l CaCl₂ x 2H₂O
1 g/l Fe(III)-Citrat;
0.2 g/l MnSO₄ x H₂0;
0.1 g/l ZnCl₂;
0.025 g/l CUSO₄ x 5H₂O,
0.02 g/l Na-Tetraborat
0.004 g/l CoCl₂ x 6H₂O
0.01 g/l Na-Molybdat.
- Prozessdauer:: 72 Stunden
- Inkubationstemperatur:: 28° C
- Rührergeschwindigkeit:: 90 UpM
- Belüftung:: 6 m³ Luft pro Stunde,

Der Fermentation wird ohne Zugabe von Antischaummittel durchgeführt. Das Produktionsmaximum wird nach ca. 40 bis 76 Stunden erreicht.

### Beispiel 5

### Herstellung der Cyclipostine X bis XV A.

Ein 200 I Fermenter wird 100 I Füllung unter folgenden Bedingungen betrieben:
- Nährmedium:: 5 g/l Glucose;
20 g/l Glycerin;
20 g/l Sojamehl;
5 g/l Hefeextrakt;
3 g/l NaCl;
2.5 ml/l Spurenelement-Lösung
pH 7.0 (vor der Sterilisation)
- Prozessdauer:: 72 Stunden,
- Inkubationstemperatur:: 27° C,
- Rührergeschwindigkeit:: 65 UpM,
- Belüftung:: 6 m³ Luft pro Stunde.

Die Fermentation wird ohne Zugabe von Mitteln zur Unterdrückung der Schaumbildung durchgeführt. Das Produktionsmaximum wird nach ca. 48 Stunden erreicht.

### Beispiel 6

### Isolierung des Cyclipostin-Gemisches aus der Kulturlösung des Streptomyces species HAG 004107, DSM 13381.

Nach Beendigung der Fermentation von *Streptomyces* species HAG 004107, DSM 13381, werden 100 Liter Kulturbrühe des Fermenters, gewonnen nach Beispiel 4, unter Zusatz von ca. 2 % Filterhilfsmittel (z.B. Celite®) filtriert und die Zellmasse (10 Liter) mit 40 Liter Methanol extrahiert. Die Wirkstoffhaltige, methanolische Lösung wird durch Filtration vom Mycel befreit und im Vakuum konzentriert. Das Konzentrat wird auf eine vorbereitete, 7 Liter ®MCl GEL, CHP20P-Säule aufgetragen. Eluiert wird mit einem Gradienten von Wasser nach Propanol-2. Der Säulendurchfluß (20 Liter pro Stunde) wird fraktioniert aufgefangen (je 10 Liter) und die Cyclipostine enthaltenden Fraktionen (19 bis 21) jeweils im Vakuum konzentriert. Mittels HPLC werden die Fraktionen untersucht (siehe Beispiel 7). Die Fraktion 19 beinhaltet das Cyclipostin A bis E sowie deren isomere, die Fraktion 20 das Cyclipostin F und Isomere hiervon, die Fraktion 21 die Inhibitoren Cyclipostin N, P, P 2, Q, R, S und T sowie ihre Isomere.

### Beispiel 7

### HPLC-Analyse der Cyclipostine.

Die Hochdruck-flüssigkeits-chromatographische (HPLC) - Analyse der Cyclipostine wird in einer HP 1100® -Anlage mit YMC-Pack Pro C18® - Säulen [AS-303, 250 x 4.6 mm, S-5 µm, 120 A°] durchgeführt. Der Durchfluß beträgt 1 ml/Minute, die Säulentemperatur 40° C. Es wird ein Gradient von 0.05% Trifluoressigsäure nach Acetonitril verwendet. 100% Acetonitril werden als Eluent nach 11 Minuten erreicht und anschließend wird mit diesem Lösungsmittel gleichbleibend (isokratisch) weiter eluiert. Die Detektion erfolgt durch Messen der Ultraviolett-Absorption bei 210 nm. Mit dieser Vorgehensweise haben die cyclipostine folgende Retentionszeiten:

| | |
|---|---|
| Cyclipostin A | 12.7 Minuten, |
| Cyclipostin A 2 | 12.6 Minuten, |
| Cyclipostin F | 13.2 Minuten, |
| Cyclipostin N | 15.9 Minuten, |
| Cyclipostin P | 17.7 Minuten, |
| Cyclipostin P 2 | 17.3 Minuten, |
| Cyclipostin Q | 18.3 Minuten, |
| Cyclipostin R | 16.7 Minuten, |
| Cyclipostin R2 | 16.4 Minuten, |
| Cyclipostin S | 18.5 Minuten, |
| Cyclipostin T | 19.1 Minuten und |
| Cyclipostin T2 | 18.7 Minuten. |

### Beispiel 8

### Darstellung von reinem Cyclipostin A und A 2.

Die Fraktion 19, erhalten nach Beispiel 6, wird im Vakuum eingeengt und 1 g des Konzentrates, gelöst in Wasser-Methanol (1:1), auf eine Nucleoprep 100-5 C₁₈ AB® Säule (21 x 250mm) aufgetragen. Eluiert wird mit einem Gradienten von 50% Acetonitril in 0.01 % Trifluoressigsäure nach 100% Acetonitril. Der Durchfluß beträgt 50 ml/Minute. Der Säulendurchfluß wird mit der Messung der Lichtabsorption bei 210 nm sowie durch Testen der Lipase-hemmenden Eigenschaften kontrolliert. Es werden Fraktionen je 60 ml abgenommen. In den Fraktionen 34 und 35 befindet sich das Cyclipostin A, in den Fraktionen 41 bis 44 das Cyclipostin A 2. Diese Fraktionen werden jeweils zusammengefaßt, im Vakuum eingeengt und nacheinander über eine SP 250/10 Nucleosil 100-5 C18 HD® - Säule aufgetrennt. Als Gradient wurde 50% bis 66% Acetonitril in 0.01 % Trifluoressigsäure gewählt, der pH-Wert der Lösungen wurde mit einem Tropfen Ammoniumhydroxyd-Lösung auf 4.0 gestellt. Die Fraktionen, die reine Verbindungen enthielten, wurden jeweils zusammengefaßt und gefriergetrocknet. Sie ergaben 5.4 mg reines Cyclipostin A als eine wachsartige Substanz und 3 mg Cyclipostin A 2 als Öl.

### Beispiel 9

Charakterisierung des Cyclipostin A.

Aussehen: in Sauerstoff enthaltenden organischen Lösungsmitteln lösliche, jedoch in Wasser und Petrolether nur wenig lösliche, neutrale, farblose, wachsartige Substanz.
UV-Maximum: 228 nm in Methanol.
IR-Banden: 1752 und 1671 cm⁻¹.
Durch hochauflösende FAB-Massenspektrometrie mit einer Nitrobenzylalkohol/LiCl-Matrix wird folgendes Molekulargewicht gefunden: 467.2757 amu, entsprechen der Summenformel für das Cyclipostinin A-Li von C₂₃H₄₁O₇PLi. Daraus ergibt sich eine Summenformel für das Cyclipostin A von C₂₃H₄₁O₇P, Molekulargewicht: 460. Durch Elektronenspray Massenspektrometrie wird im positivem lonisations-Modus (ES), positiv), ein Peak bei 461 amu, entsprechend (M + H)⁺ gefunden; darüber hinaus der charakteristische Peak bei 221 amu, entsprechend C₇H₁₀O₆P. Im ESInegativ - Modus findet man 459 amu (M - H)⁻, 337 amu (C₁₆H₃₄O₅P) und 219 amu (C₇H₈O₆P). Zur Bestimmung der Position der Alkohol-Gruppe wird mit N-Methyl-N-trimethylsilyl-trifluoracetamid derivatisiert und die Probe mit Elekronen-lonisations - Massenspektrometrie untersucht. Es entsteht das Trimethylsilyl - Derivat: 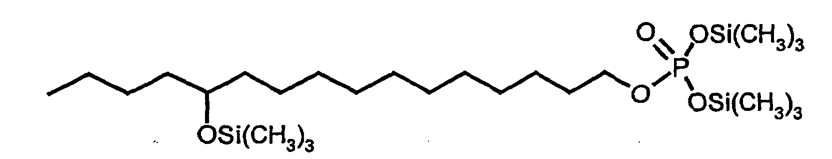 der Masse 554 amu. Die Position der silylierten Hydroxygruppe wird durch die intensiven lonen bei 497 amu (α-Spaltung) und 159 amu (α-Spaltung) angezeigt. NMR-Signale: siehe Tabelle 1.

**Tabelle 1:**

| ¹H- und ¹³C- chemische Verschiebungen von Cyclipostin A in Methanol-d₄ bei 300K | | |
|---|---|---|
| | ¹H | ¹³C |
| 1 | - | 171.08 (1.4 Hz)^{b)} |
| 2 | - | 114.61 (3.4 HZ)^{b)} |
| 3 | 3.87 | 40.75 |
| 4 | 4.46/3.86 | 66.04 |
| 5 | 4.31/4.25 | 69.39 (6.0 HZ)^{b)} |
| 6 | - | 161.47 (8.0 HZ)^{b)} |
| 7 | 2.40 | 17.89 (4.6 HZ)^{b)} |
| 1' | 4.25 | 71.61 (6.6 HZ)^{b)} |
| 2' | 1.73 | 31.16 (6.6 HZ)^{b)} |
| 3' | 1.41 | 26.39 |
| n' | 3.49 | 72.45 |
| n±1 | 1.46-1.33 | 38.44,38.15 |
| 4'-14' (a) | 1.37-1.26 | 30.85-30.58 |
| 15' | 1.34 | 23.84 |
| 16' | 0.91 | 14.43 |

| | | |
|---|---|---|
| a) außer n und n±1. | | |
| b) in Klammem sind die ¹³C/³¹P-Kopplungskonstanten angegeben. | | |

### Beispiel 10

### Charakterisierung des Cyclipostin B.

Cyclipostin B wird wie im Beispiel 8 für Cyclipostin A beschrieben, durch mehrfache Wiederholung der chromatographischen Schritte isoliert und wie im Beispiel 9 Charakterisiert.
Aussehen: in Sauerstoff enthaltenden organischen Lösungsmitteln lösliche, jedoch
in Wasser und Petrolether nur wenig lösliche, neutrale, farblose, wachsartige Substanz.
UV-Maximum: 228 nm in Methanol.
Durch Elektronenspray Massenspektrometrie wird im positivem lonisations-Modus (ESI, positiv) ein Peak bei 461 amu, entsprechend (M + H)⁺ gefunden; darüber hinaus der charakteristischen Peak bei 221 amu, entsprechend C₇H₁₀O₆P. Im ESInegativ - Modus findet man 459 amu (M - H)⁻, 337 amu (C₁₆H₃₄O₅P) und 219 amu (C₇H₈O₆P). Zur Bestimmung der Position der Alkohol-Gruppe wird mit N-Methyl-N-trimethylsilyl-trifluoracetamid derivatisiert und die Probe mit Elekronen-lonisations - Massenspektrometrie untersucht. Es entsteht das Trimethylsilyl - Derivat der Masse 554 amu:

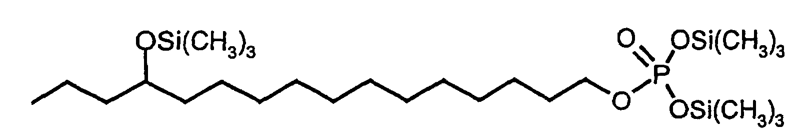

Die Position der silylierten Hydroxygruppe wird durch die intensiven Ionen bei 511 amu (α-Spaltung) und 145 amu (α-Spaltung) angezeigt.

Summenformel des Cyclipostin B: C₂₃H₄₁O₇P, Molekulargewicht: 460.

### Beispiel 11

### Charakterisierung des Cyclipostin C.

Cyclipostin C wird wie im Beispiel 8 für Cyclipostin A beschrieben, durch mehrfache wiederholung der chromatographischen Schritte isoliert und wie im Beispiel 9 Charakterisiert.
Aussehen: in Sauerstoff enthaltenden organischen Lösungsmitteln lösliche, jedoch in Wasser und Petrolether nur wenig lösliche, neutrale, farblose, wachsartige Substanz.
UV-Maximum: 228 nm in Methanol.
Durch Elektronenspray Massenspektrometrie wird im positivem lonisations-Modus (ESI, positiv) ein Peak bei 461 amu, entsprechend (M + H)⁺ gefunden; darüber hinaus der charakteristischen Peak bei 221 amu, entsprechend C₇H₁₀O₆P. Im ESInegativ - Modus findet man 459 amu (M - H)⁻, 337 amu (C₁₆H₃₄O₅P) und 219 amu (C₇H₈O₆P). Zur Bestimmung der Position der Alkohol-Gruppe wird mit N-Methyl-N-trimethylsilyl-trifluoracetamid derivatisiert und die Probe mit Elekronen-Ionisations - Massenspektrometrie untersucht. Es entsteht das Trimethylsilyl - Derivat der Masse 554 amu:

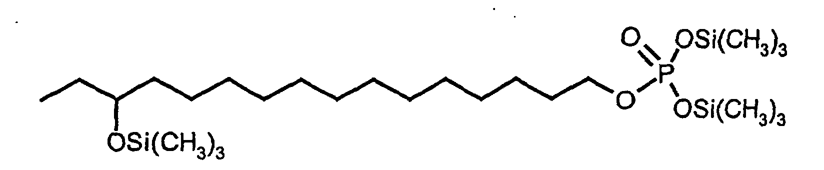

Die Position der silylierten Hydroxygruppe wird durch die intensieven lonen bei 525 amu (α-Spaltung) und 131 amu (α-Spaltung) angezeigt.

Summenformel des Cyclipostin C: C₂₃H₄₁O₇P, Molekulargewicht: 460.

### Beispiel 12

### Charakterisierung des Cyclipostin F.

Die Fraktion 20, gewonnen nach Beispiel 6, wird wie in Beispiel 8 beschrieben, aufgetrennt und das Cyclipostin F durch mehrfache wiederholung der chromatographischen Schritte isoliert und wie im Beispiel 9 Charakterisiert. Retentionszeit: 13.2 Minuten.
Aussehen: in Sauerstoff enthaltenden organischen Lösungsmitteln lösliche, jedoch in Wasser und Petrolether nur wenig lösliche, neutrale, farblose, wachsartige Substanz.
UV-Maximum: 228 nm in Methanol.
Durch Elektronenspray Massenspektrometrie wird im positivem lonisations-Modus (ESI, positiv) ein Peak bei 459 amu, entsprechend (M + H)⁺ gefunden; darüber hinaus der charakteristischen Peak bei 221 amu, entsprechend C₇H₁₀O₆P. Im ESInegativ - Modus findet man 457.6 amu (M - H)⁻, 336 amu (C₁₆H₃₂O₅P) und 219 amu (C₇H₈O₆P).
Summenformel des Cyclipostin F: C₂₃H₃₉O₇P, Molekulargewicht: 458.

### Beispiel 13

Charakterisierung des Cyclipostin P.

Die Fraktion 21, gewonnen nach den Beispielen 5 und 6, wird wie in Beispiel 8 beschrieben, aufgetrennt und das Cyclipostin P durch mehrfache wiederholung der chromatographischen Schritte isoliert (210 mg) und wie im Beispiel 9 charakterisiert. Durch Auflösen der 210 mg in 3 ml Propanol-2 und 13 ml Acetonitril und Zugabe von 8 ml Wasser wurde das Cyclipostin P kristallisiert. Nach Abfiltrieren, Waschen mit kaltem Acetonitril wurden 135 mg Cyclipostin ausgewogen. Fp. 58-59° C. Retentionszeit: 17.7 Minuten.
Aussehen: in Sauerstoff enthaltenden organischen Lösungsmitteln lösliche, jedoch in Wasser und Petrolether nur wenig lösliche, neutrale, farblose, wachsartige Substanz.
UV-Maximum: 228 nm in Methanol.
IR-Banden: 2917, 2852, 1753, 1671, 1471, 1214, 996 und 832 cm⁻¹.
Durch hochauflösende FAB-Massenspektrometrie mit einer Nitrobenzylalkohol-Matrix wird folgendes Molekulargewicht gefunden: 445.2717 amu, entsprechen dem (M + H)⁺ für das Cyclipostin P von C₂₃H₄₂O₆P. Daraus ergibt sich eine Summenformel für das Cyclipostin P von C₂₃H₄₁O₆P, Molekulargewicht:444. Durch Elektronenspray Massenspektrometrie wird im positivem Ionisations-Modus (ESI, positiv) ein Peak bei 445 amu, entsprechend (M + H)⁺ gefunden; darüber hinaus der charakteristischen Peak bei 221 amu, entsprechend C₇H₁₀O₆P. Im ESlnegativ - Modus findet man 443 amu (M - H)⁻, 321 amu (C₁₆H₃₄O₄P) und 219 amu (C₇H₈O₆P).

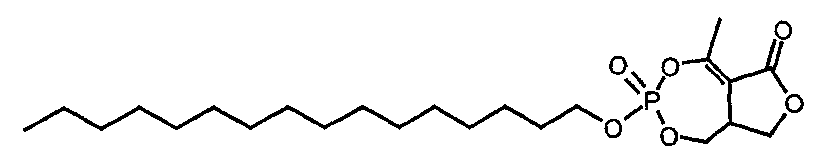

Die NMR-Daten sind in Tabelle 2 wiedergegeben.

**Tabelle 2.**

| Chemische Verschiebungen von Cyclipostatin P in MeOD bei 300 K. | | |
|---|---|---|
| | ¹H | ¹³C |
| 1 | - | 171.08 |
| 2 | - | 114.60 (3.0 Hz)^{a)} |
| 3 | 3.87 | 40.74 |
| 4 | 4.47/3.85 | 66.05 |
| 5 | 4.30/4.25 | 69.40 (6.0 Hz)^{a)} |
| 6 | - | 161.47 (8.0 Hz)^{a)} |
| 7 | 2.40 | 17.90 (4.6 Hz)^{a)} |
| 1' | 4.24 | 71.62 (6.9 Hz)^{a)} |
| 2' | 1.73 | 31.16 (6.3 Hz)^{a)} |
| 3' | 1.41 | 26.38 |
| 4'-13' | 1.34-1.29 | 30.76-30.11 |
| 14' | 1.34-1.29 | 33.07 |
| 15' | 1.31 | 23.72 |
| 16' | 0.89 | 14.42 |

| | | |
|---|---|---|
| a) in Klammem sind die ¹³C/³¹-Kopplungskonstanten angegeben. | | |

### Beispiel 14

### Charakterisierung des Cyclipostin P 2.

Die Fraktion 21, gewonnen nach Beispiel 5 und 6, wird wie in Beispiel 8 beschrieben, aufgetrennt und das Cyclipostin P 2 durch mehrfache Wiederholung der chromatographischen Schritte isoliert (130 mg) und wie im Beispiel 9 charakterisiert.
Retentionszeit: 17.1 Minuten.
Aussehen: in Sauerstoff enthaltenden organischen Lösungsmitteln lösliche, jedoch in Wasser und Petrolether nur wenig lösliche, neutrale, farblose, ölige Substanz. UV-Maximum: 228 nm in Methanol.
Durch hochauflösende FAB-Massenspektrometrie mit einer Nitrobenzylalkohol-Matrix wird folgendes Molekulargewicht gefunden: 445.2721 amu, entsprechen dem (M + H)⁺ für das Cyclipostin P von C₂₃H₄₂O₆P. Daraus ergibt sich eine Summenformel für das Cyclipostin P 2 von C₂₃H₄₁O₆P, Molekulargewicht 444. Durch Elektronenspray Massenspektrometrie wird im positivem lonisations-Modus (ESI, positiv) ein Peak bei 445 amu, entsprechend (M + H)⁺ gefunden; darüber hinaus der charakteristischen Peak bei 221 amu, entsprechend C₇H₁₀O₆P. Im ESInegativ - Modus findet man 443 amu (M - H)⁻, 321 amu (C₁₆H₃₄O₄P) und 219 amu (C₇H₈O₆P).

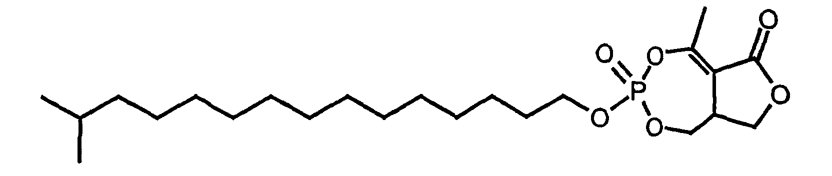

Die NMR-Daten des Cyclipostin P 2 sind in Tabelle 3 wiedergegeben.

**Tabelle 3**

| Chemische Verschiebungen von Cyclipostin P 2 in CD₃OD bei 300 K. | | |
|---|---|---|
| | ¹H | ¹³C |
| 1 | - | 171.05 |
| 2 | - | 114.60 (3.2 Hz)^{a}) |
| 3 | 3.87 | 40.74 |
| 4 | 4.46/3.85 | 66.02 |
| 5 | 4.30/4.25 | 69.38 (6.0 Hz)^{a} |
| 6 | - | 161.46 (8.0 Hz)^{a} |
| 7 | 2.40 | 17.90 (4.6 Hz)^{a} |
| 1' | 4.24 | 71.60 (6.9 Hz)^{a} |
| 2' | 1.73 | 31.16 (6.3 Hz)^{a} |
| 3' | 1.41 | 26.39 |
| 4'-11' | 1.34-1.29 | 31.04-30.11 |
| 12' | 1.29 | 28.53 |
| 13' | 1.17 | 40.25 |
| 14' | 1.52 | 29.15 |
| 15'. 16' | 0.87 | 23.04 |

| | | |
|---|---|---|
| a) in Klammem sind die ¹³C^{31p}-Kopplungskonstanten angegeben. | | |

### Beispiel 15

### Gewinnung und Charakterisierung des Cyclipostin N.

Die Fraktion 21, gewonnen nach den Beispielen 5 und 6, wird wie in Beispiel 8 beschrieben, aufgetrennt und das Cyclipostin N durch mehrfache wiederholung der chromatographischen Schritte isoliert (2 mg) und wie im Beispiel 9 charakterisiert.

Retentionszeit: 15.9 Minuten.
Aussehen: in Sauerstoff enthaltenden organischen Lösungsmittel lösliche, jedoch in Wasser und Petrolether nur wenig lösliche, neutrale, farblose, ölige Substanz. UV-Maximum: 228 nm in Methanol.
Durch hochauflösende Massenspektrometrie FAB-Bedingungen wurde ein Quasi-Molekülion (M + H) bei 417.2405 beobachtet entsprechend einer Summenformel von C₂₁H₃₈O₆P (Theorie: 417.2406). Charakteristisches Fragment im ESI⁺-Modus: 221 amu.

**Tabelle 4:**

| Chemische Verschiebungen von Cyclipostin N in MeOD bei 300 K. | | |
|---|---|---|
| | ¹H | ¹³C |
| 1 | - | 171.07 |
| 2 | - | 114.60 (3.1 HZ)^{a)} |
| 3 | 3.87 | 40.74 |
| 4 | 4.45/3.84 | 66.03 |
| 5 | 4.30/4.25 | 69.39 (5.9 Hz)^{a)} |
| 6 | - | 161.47 (8.0 Hz)^{a)} |
| 7 | 2.40 | 17.90 (4.9 Hz)^{a)} |
| 1' | 4.24 | 71.60 (6.6 Hz)^{a)} |
| 2' | 1.73 | 31.16 (6.2 Hz)^{a)} |
| 3' | 1.41 | 26.38 |
| 4'-11' | 1.35-1.26 | 30.76-30.11 |
| 12' | 1.35-1.26 | 33.06 |
| 13' | 1.31 | 23.72 |
| 14' | 0.89 | 14.41 |

| | | |
|---|---|---|
| a) in Klammem sind die¹³C/³¹P-Kopplungskonstanten angegeben. | | |

### Beispiel 16

### Gewinnung und Charakterisierung des Cyclipostin R.

Die Fraktion 21, gewonnen nach den Beispielen 5 und 6, wird wie in Beispiel 8 beschrieben, aufgetrennt und das Cyclipostin R durch mehrfache Wiederholung der chromatographischen Schritte isoliert (8 mg) und wie im Beispiel 9 charakterisiert. Retentionszeit: 16.7 Minuten.
Aussehen: in Sauerstoff enthaltenden organischen Lösungsmitteln lösliche, jedoch in Wasser und Petrolether nur wenig lösliche, neutrale, farblose, kristalline Substanz. UV-Maximum: 228 nm in Methanol.
Durch hochauflösende Massenspektrometrie FAB-Bedingungen wurde ein Quasi-Molekülion (M + H) bei 431.2561 beobachtet entsprechend einer Summenformel von C₂₂H₄₀O₆P (Theorie: 431.2562). Charakteristisches Fragment im ESI⁺-Modus: 221 amu.

**Tabelle 5:**

| Chemische Verschiebungen von Cyclipostin R in MeOD bei 300 K. | | |
|---|---|---|
| | ¹H | ¹³C |
| 1 | - | 171.06 |
| 2 | - | 114.58 (3.2 Hz)^{a)} |
| 3 | 3.87 | 40.75 |
| 4 | 4.45/3.85 | 66.04 |
| 5 | 4.30/4.25 | 69.40 (6.0 HZ)^{a)} |
| 6 | - | 161.48 (8.0 Hz)^{a)} |
| 7 | 2.40 | 17.90 (5.0 HZ)^{a)} |
| 1' | 4.24 | 71.61 (7.0 Hz)^{a)} |
| 2' | 1.73 | 31.16 (6.2 HZ)^{a)} |
| 3' | 1.41 | 26.38 |
| 4'-12' | 1.37-1.25 | 30.74-30.10 |
| 13' | 1.17 | 33.06 |
| 14' | 1.30 | 23.71 |
| 15' | 0.89 | 14.40 |

| | | |
|---|---|---|
| a) in Klammem sind die ¹³C/³¹P-Kopplungskonstanten angegeben. | | |

### Beispiel 17

### Gewinnung und Charakterisierung des Cyclipostin R2.

Die Fraktion 21, gewonnen nach den Beispielen 5 und 6, wird wie in Beispiel 8 beschrieben, aufgetrennt und das Cyclipostin R2 durch mehrfache wiederholung der chromatographischen Schritte isoliert (8 mg) und wie im Beispiel 9 charakterisiert. Retentionszeit: 16.4 Minuten.
Aussehen: in Sauerstoff enthaltenden organischen Lösungsmitteln lösliche, jedoch in Wasser und Petrolether nur wenig lösliche, neutrale, farblose, ölige Substanz. UV-Maximum: 228 nm in Methanol.
Durch hochauflösende Massenspektrometrie FAB-Bedingungen wurde ein Quasi-Molekülion (M + H) bei 431.2564 beobachtet entsprechend einer Summenformel von C₂₂H₄₀O₆P (Theorie: 431.2562). Charakteristisches Fragment im ESI⁺-Modus: 221 amu.

**Tabelle 6.**

| Chemische Verschiebungen von Cyclipostin R2 in MeOD bei 300 K. | | |
|---|---|---|
| | ¹H | ¹³C |
| 1 | - | 171.06 (1.7 HZ)^{a)} |
| 2 | - | 114.58 (3.1 Hz)^{a)} |
| 3 | 3.87 | 40.75 |
| 4 | 4.46/3.85 | 66.03 |
| 5 | 4.30/4.25 | 69.39 (6.0 Hz)^{a)} |
| 6 | - | 161.47 (8.0 Hz)^{a)} |
| 7 | 2.40 | 17.90 (4.9 Hz)^{a)} |
| 1' | 4.24 | 71.60 (6.9 Hz)^{a)} |
| 2' | 1.73 | 31.16 (6.6 Hz)^{a)} |
| 3' | 1.41 | 26.38 |
| 4'-10' | 1.37-1.25 | 31.02-30.10 |
| 11' | 1.29 | 28.51 |
| 12' | 1.16 | 40.24 |
| 13' | 1.51 | 29.15 |
| 14'. 15' | 0.87 | 23.02 |

| | | |
|---|---|---|
| a) in Klammem sind die ¹³C/³¹P-Kopplungskonstanten angegeben. | | |

### Beispiel 18

### Gewinnung und Charakterisierung des Cyclipostin S.

Die Fraktion 21, gewonnen nach den Beispielen 5 und 6, wird wie in Beispiel 8 beschrieben, aufgetrennt und das Cyclipostin S durch mehrfache Wiederholung der chromatographischen Schritte isoliert (0.7 mg) und wie im Beispiel 9 charakterisiert. Retentionszeit: 18.5 Minuten.
Aussehen: in Sauerstoff enthaltenden organischen Lösungsmitteln lösliche, jedoch in Wasser und Petrolether nur wenig lösliche, neutrale, farblose, feste Substanz.
UV-Maximum: 228 nm in Methanol.
Durch hochauflösende Massenspektrometrie FAB-Bedingungen wurde ein Quasi-Molekülion (M + H) bei 459.2883 beobachtet entsprechend einer Summenformel von C₂₄H₄₄O₆P (Theorie: 459.2575). Charakteristisches Fragment im ESI⁺-Modus: 235 amu.

**Tabelle 7.**

| Chemische Verschiebungen von Cyclipostin S in MeOD bei 300 K. | | |
|---|---|---|
| | ¹H | ¹³C |
| 1 | - | 170.87 (1.4 Hz)^{a)} |
| 2 | - | 113.66 (3.1 HZ)^{a)} |
| 3 | 3.85 | 40.77 |
| 4 | 4.45/3.85 | 66.04 |
| 5 | 4.29/4.24 | 69.17 (6.0 HZ)^{a)} |
| 6 | - | 165.80 (8.3 Hz)^{a)} |
| 7 | 2.98/2.82 | 25.05 (4.6 Hz)^{a)} |
| 8 | 1.16 | 10.86 |
| 1' | 4.25 | 71.57 (6.9 Hz)^{a)} |
| 2' | 1.74 | 31.19 (6.3 Hz)^{a)} |
| 3' | 1.42 | 26.41 |
| 4'-13' | 1.34-1.29 | 30.76-30.11 |
| 14' | 1.34-1.29 | 33.07 |
| 15' | 1.31 | 23.73 |
| 16' | 0.89 | 14.43 |

| | | |
|---|---|---|
| a) in Klammem sind die ¹³C/³¹P-Kopplungskonstanten angegeben. | | |

### Beispiel 19

### Gewinnung und Charakterisierung des Cyclipostin T.

Die Fraktion 21, gewonnen nach den Beispielen 5 und 6, wird wie in Beispiel 8 beschrieben, aufgetrennt und das Cyclipostin T durch mehrfache Wiederholung der chromatographischen Schritte isoliert (5 mg) und wie im Beispiel 9 charakterisiert.
Retentionszeit: 19.1 Minuten.
Aussehen: in Sauerstoff enthaltenden organischen Lösungsmitteln lösliche, jedoch in Wasser und Petrolether nur wenig lösliche, neutrale, farblose, feste Substanz. UV-Maximum: 228 nm in Methanol.
Durch hochauflösende Massenspektrometrie FAB-Bedingungen wurde ein Quasi-Molekülion (M + H) bei 473.3030 beobachtet entsprechend einer Summenformel von C₂₅H₄₆O₆P (Theorie: 473.3032). Charakteristisches Fragment im ESI⁺-Modus: 249 amu.

**Tabelle 8.**

| Chemische Verschiebungen von Cyclipostin T in MeOD bei 300 K. | | |
|---|---|---|
| | ¹H | ¹³C |
| 1 | - | 170.98 (1.7 Hz)^{a)} |
| 2 | - | 114.39 (3.1 Hz)^{a)} |
| 3 | 3.87 | 40.78 |
| 4 | 4.46/3.85 | 66.02 |
| 5 | 4.2914.26 | 69.23 (5.9 Hz)^{a)} |
| 6 | - | 164.69 (8.7 Hz)^{a)} |
| 7 | 2.89/2.83 | 33.35 (4.5 Hz)^{a)} |
| 8 | 1.65 | 20.63 |
| 9 | 0.98 | 13.84 |
| 1' | 4.25 | 71.57 (6.6 Hz)^{a)} |
| 2' | 1.74 | 31.18 (6.2 Hz)^{a)} |
| 3' | 1.42 | 26.42 |
| 4'-13' | 1.34-1.29 | 30.78-30.11 |
| 14' | 1.34-1.29 | 33.06 |
| 15' | 1.31 | 23.72 |
| 16' | 0.89 | 14.42 |

| | | |
|---|---|---|
| a) in Klammem sind die ¹³C/³¹ P-Kopplungskonstanten angegeben. | | |

### Beispiel 20

Gewinnung und Charakterisierung des Cyclipostin T2.
Die Fraktion 21, gewonnen nach den Beispielen 5 und 6, wird wie in Beispiel 8 beschrieben, aufgetrennt und das Cyclipostin T2 durch mehrfache Wiederholung der chromatographischen Schritte isoliert (4 mg) und wie im Beispiel 9 charakterisiert.
Retentionszeit: 18.7 Minuten.
Aussehen: in Sauerstoff enthaltenden organischen Lösungsmitteln lösliche, jedoch in Wasser und Petrotether nur wenig lösliche, neutrale, farblose, feste Substanz. UV-Maximum: 228 nm in Methanol.
Durch hochauflösende Massenspektrometrie FAB-Bedingungen wurde ein Quasi-Molekülion (M + H) bei 473.3035 beobachtet entsprechend einer Summenformel von C₂₅H₄₆O₆P (Theorie: 473.3032). Charakteristisches Fragment im ESI⁺-Modus: 249 amu.

**Tabelle 9.**

| Chemische Verschiebungen von Cyclipostin T2 in MeOD bei 300 K. | | |
|---|---|---|
| | ¹H | ¹³C |
| 1 | - | 170.98 (1.7 HZ)^{a)} |
| 2 | - | 114.40 (3.1 Hz)^{a)} |
| 3 | 3.87 | 40.78 |
| 4 | 4.46/3.85 | 66.02 |
| 5 | 4.29/4.25 | 69.23 (5.9 HZ)^{a)} |
| 6 | - | 164.69 (8.7 Hz)^{a)} |
| 7 | 2.90/2.83 | 33.35 (4.5 Hz)^{a)} |
| 8 | 1.65 | 20.63 |
| 9 | 0.98 | 13.84 |
| 1' | 4.24 | 71.57 (6.9 HZ)^{a)} |
| 2' | 1.74 | 31.18 (6.2 HZ)^{a)} |
| 3' | 1.42 | 26.42 |
| 4'-11' | 1.37-1.25 | 31.03-30.11 |
| 12' | 1.29 | 28.52 |
| 13' | 1.17 | 40.25 |
| 14' | 1.52 | 29.15 |
| 15',16' | 0.87 | 23.03 |

| | | |
|---|---|---|
| a) in Klammern sind die ¹³C/³¹P-Kopptungsf<onstanten angegeben. | | |

### Beispiel 21

### Hemmung der hormon-sensitiven Lipase (HSL).

Die hormon-sensitive Lipase aus Ratten wird mit Trioleyl-glycerin als Substrat in folgenden Konzentrationen gehemmt (IC₅₀):

| | |
|---|---|
| Cyclipostin A | 20 nM, |
| Cyclipostin N | 450 nM, |
| Cyclipostin P | 30 nM, |
| Cyclipostin P2 | 40 nM, |
| Cyclipostin R | 10 nM, |
| Cyclipostin R2 | 220 nM, |
| Cyclipostin S | 20 nM, |
| Cyclipostin T | 200 nM, |
| Cyclipostin T2 | 60 nM. |

## Patentansprüche

1. Verbindung der allgemeinen Formel I 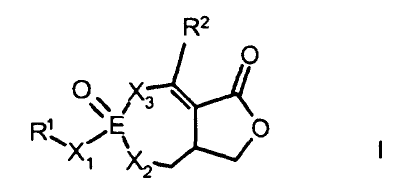 worin
R¹ für
1. eine Kohlenstoff-Kette mit 10 bis 18 C - Atomen, die geradkettig, verzweigt, gesättigt oder ungesättigt, carbo- oder heterocyclisch sein kann und worin die Kohlenstoff-Kette gegebenenfalls ein- oder zweifach substituiert ist durch
1.1 -OH,
1.2 =O,
1.3 -O-C₁-C₆-Alkyl, worin Alkyl gerade oder verzweigt ist,
1.4 -O-C₂-C₆-Alkenyl, worin Alkenyl gerade oder verzweigt ist,
1.5 -C₁-C₆-Alkyl, worin Alkyl gerade oder verzweigt ist,
1.6 - Aryl,
1.7 -C₁-C₆-Alkylbenzol,
1.8 . - Diphenyl,
1.9 -NH-C₁-C₆-Alkyl, worin Alkyl gerade oder verzweigt ist,
1.10 -NH-C₂-C₆-Alkenyl, worin Alkenyl gerade oder verzweigt ist,
1.11 -NH₂,
1.12 =S,
1.13 -S-C₁-C₆-Alkyl, worin Alkyl gerade oder verzweigt ist, oder
1.14 -S-C₂-C₆-Atkenyl, worin Alkenyl gerade oder verzweigt ist,
1.15 Halogen,
worin die Substituenten 1.1 bis 1.15 auch noch substituiert sein können, steht;
R² für
1.0 C₁-C₆-Alkyl, worin Alkyl unsubstituiert oder ein- oder zweifach wie unter 1.1 bis 1.15 beschrieben substituiert ist,
2.0 C₂-C₆-Alkenyl, worin Alkenyl unsubstituiert oder ein- oder zweifach wie unter 1.1 bis 1.15 beschrieben substituiert ist,
3.0 C₂-C₆-Alkinyl, worin Alkinyl unsubstituiert oder ein- oder zweifach wie unter 1.1 bis 1.15 beschrieben substituiert ist ,steht,
E für Phosphor- (P) und
X₁, X₂ und X₃ unabhängig voneinander für -O- stehen,
in allen ihren stereochemischen Formen und Gemische dieser Formen in jedem Verhältnis, sowie deren physiologisch verträglichen Salzen.

2. Verbindung der Formel I oder ein physiologisch verträgliches Salz davon gemäß Anspruch 1, **dadurch gekennzeichnet, daß**
R¹ für
1.0 -(CH₂)₁₅CH₃,
2.0 -(CH₂)₁₃CH(CH₃)₂,
3.0 -(CH₂)₁₁CH(OH)(CH₂)₃CH₃.
4.0 -(CH₂)₁₁CH(OH)CH₂CH(CH₃)₂,
5.0 -(CH₂)₁₂CH(OH)(CH₂)₂CH₃,
6.0 -(CH₂)₁₃CH(OH)CH₂CH₃,
7.0 -(CH₂)₁₄CH(OH)CH₃,
8.0 -(CH₂)₁₅CH₂(OH),
9.0 -(CH₂)₁₆CH₃,
10.0 -(CH₂)₁₃C=OCH₂CH₃
11.0 -(CH₂)₁₂C=OCH₂CH₂CH₃
12.0 -(CH₂)₁₁C=OCH₂CH₂CH₂CH₃
13.0 -(CH₂)₁₃CH₃
14.0 -(CH₂)₁₁CH(CH₃)₂
15.0 -(CH₂)₁₄CH₃ oder
16.0 -(CH₂)₁₂CH(CH₃)₂
steht.

3. Verbindung der Formel I oder ein physiologisch verträgliches Salz davon gemäß Anspruch 1 oder 2, **dadurch gekennzeichnet, daß** R² für C₁-C₆-Alkyl steht.

4. Verbindung der Formel I oder ein physiologisch verträgliches Salz davon gemäß Anspruch 3, **dadurch gekennzeichnet daß** R² für -CH₃, -CH₂CH₃ oder -CH₂CH₂CH₃ steht.

5. Verbindung der Formel I oder ein physiologisch verträgliches Salz davon gemäß einem oder mehreren der Ansprüche 1 bis 4, herstellbar indem der Mikroorganismus *Streptomyces* species HAG 004107, DSM 13381, oder einer seiner Varianten oder Mutanten unter geeigneten Bedingungen in einem Kulturmedium fermentiert wird, bis sich eine oder mehrere Verbindungen der allgemeinen Formel I in dem Kulturmedium anhäufen und anschließend aus dem Kulturmedium isoliert und gegebenenfalls in chemische Äquivalente und physiologisch verträgliche Salze überführt werden.

6. Verfahren zur Herstellung einer Verbindung der Formel I oder ein physiologisch verträgliches Salz davon gemäß einem oder mehreren der Ansprüche 1-5, **dadurch gekennzeichnet, daß** der Mikroorganismus *Streptomyces* species HAG 004107, DSM 13381, oder einer seiner Varianten oder Mutanten unter geeigneten Bedingungen in einem Kulturmedium fermentiert wird, bis sich eine oder mehrere Verbindungen der allgemeinen Formel I in dem Kulturmedium anhäufen und anschließend aus dem Kulturmedium isoliert und gegebenenfalls in chemische Äquivalente und physiologisch verträgliche Salze überführt werden.

7. Verfahren gemäß Anspruch 6, **dadurch gekennzeichnet, daß** die Fermentation unter aeroben Bedingunen bei einer Temperatur zwischen 18 und 35°C und bei einem pH zwischen 6 und 8 durchgeführt wird.

8. Verbindung der Formel I oder ein physiologisch verträgliches Salz davon gemäß einem oder mehreren der Ansprüche 1-5 zur Verwendung als Arzneimittel.

9. Verbindung der Formel I oder ein physiologisch verträgliches Salz davon gemäß einem oder mehreren der Ansprüche 1-5 zur Verwendung als Arzneimittel zur Hemmung von Lipasen.

10. Verbindung der Formel I oder ein physiologisch verträgliches Salz davon gemäß einem oder mehreren der Ansprüche 1-5 zur Verwendung als Antidiabetikum.

11. Pharmazeutische Zubereitung mit einem Gehalt an mindestens einer Verbindung der Formel I oder ein physiologisch verträgliches Salz davon gemäß einem oder mehreren der Ansprüche 1-5.

12. Verfahren zur Herstellung von pharmazeutischen Zubereitungen gemäß Anspruch 11, **dadurch gekennzeichnet, daß** man mindestens eine Verbindung der Formel I oder ein verträgliches Salz davon gemäß einem oder mehreren der Ansprüche 1-5 mit geeigneten Hilfs- und/oder Trägerstoffen in eine geeignete Darreichungsform bringt.

13. *Streptomyces* species HAG 004107, DSM 13381.

## Claims

1. A compound of the formula I 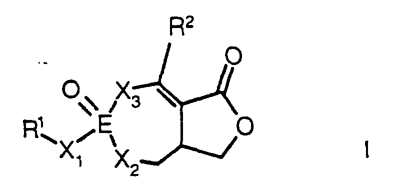 in which
R¹ is
1. a carbon chain having 10 to 18 carbon atoms, which can be straight-chain, branched, saturated or unsaturated, carbo- or heterocyclic and in which the carbon chain is optionally mono- or disubstituted by:
1.1 -OH,
1.2 =O,
1.3 -O-C₁-C₆-alkyl, in which alkyl is linear or branched,
1.4 -O-C₂-C₆-alkenyl, in which alkenyl is linear or branched,
1.5 -C₁-C₆-alkyl, in which alkyl is linear or branched,
1.6 - aryl,
1.7 -C₁-C₆-alkylbenzene,
1.8 - diphenyl,
1.9 -NH-C₁-C₆-alkyl, in which alkyl is linear or branched,
1.10 -NH-C₂-C₆-alkenyl, in which alkenyl is linear or branched,
1.11 -NH₂,
1.12 =S,
1.13 -S-C₁-C₆-alkyl, in which alkyl is linear or branched, or
1.14 -S-C₂-C₆-alkenyl, in which alkenyl is linear or branched,
1.15 halogen,
in which the substituents 1.1 to 1.15 can also be additionally substituted;
R² is
1.0 C₁-C₆-alkyl, in which alkyl is unsubstituted or mono- or disubstituted as described in 1.1 to 1.15,
2.0 C₂-C₆-alkenyl, in which alkenyl is unsubstituted or mono- or disubstituted as described in 1.1 to 1.15, or,
3.0 C₂-C₆-alkynyl, in which alkynyl is unsubstituted or mono- or disubstituted as described in 1.1 to 1.15,
E is a phosphorus (P) atom, and
X₁, X₂ and X₃ independently of one another are -O-,
in all its stereochemical forms and mixtures of these forms in any ratio, or its physiologically tolerable salts.

2. A compound of the formula I or a physiologically tolerable salt thereof as claimed in claim 1 wherein
R¹ is
1.0 -(CH₂)₁₅CH₃,
2.0 -(CH₂)₁₃CH(CH₃)₂,
3.0 -(CH₂)₁₁CH(OH)(CH₂)₃CH₃,
4.0 -(CH₂)₁₁CH(OH)CH₂CH(CH₃)₂,
5.0 -(CH₂)₁₂CH(OH)(CH₂)₂CH₃,
6.0 -(CH₂)₁₃CH(OH)CH₂CH₃,
7.0 -(CH₂)₁₄CH(OH)CH₃,
8.0 -(CH₂)₁₅CH₂(OH),
9.0 -(CH₂)₁₆CH₃,
10.0 -(CH₂)₁₃C=OCH₂CH₃
11.0 -(CH₂)₁₂C=OCH₂CH₂CH₃
12.0 -(CH₂)₁₁C=OCH₂CH₂CH₂CH₃
13.0 -(CH₂)₁₃CH₃
14.0 -(CH₂)₁₁CH(CH₃)₂
15.0 -(CH₂)₁₄CH₃ or
16.0 -(CH₂)₁₂CH(CH₃)₂.

3. A compound of the formula I or a physiologically tolerable salt thereof as claimed in claim 1 or 2, wherein R² is C₁-C₆-alkyl.

4. A compound of the formula I or a physiologically tolerable salt thereof as claimed in claim 3, wherein R² is -CH_{3.} -CH₂CH₃ or -CH₂CH₂CH₃.

5. A compound of the formula I or a physiologically tolerable salt thereof as claimed in one or more of claims 1 to 4, preparable by fermenting the microorganism *Streptomyces* species HAG 004107, DSM 13381, or one of its variants or mutants under suitable conditions in a culture medium until one or more compounds of the formula I accumulate in the culture medium and then isolating them from the culture medium and optionally converting them into chemical equivalents or physiologically tolerable salts.

6. A process for the preparation of a compound of the formula I or a physiologically tolerable salt thereof as claimed in one or more of claims 1-5, which comprises fermenting the microorganism Streptomyces species HAG 004107, DSM 13381, or one of its variants or mutants under suitable conditions in a culture medium until one or more compounds of the formula I accumulates in the culture medium and then isolating them from the culture medium and optionally converting them into chemical equivalents or physiologically tolerable salts.

7. The process as claimed in claim 6, wherein the fermentation is carried out under aerobic conditions at a temperature between 18 and 35°C and at a pH between 6 and 8.

8. A compound of the formula I or a physiologically tolerable salt thereof as claimed in one or more of claims 1-5 for use as a pharmaceutical.

9. A compound of the formula I or a physiologically tolerable salt thereof as claimed in one or more of claims 1-5 for use as a pharmaceutical for the inhibition of lipases.

10. A compound of the formula I or a physiologically tolerable salt thereof as claimed in one or more of claims 1-5 for use as an antidiabetic.

11. A pharmaceutical preparation which contains at least one compound of the formula I or a physiologically tolerable salt thereof as claimed in one or more of claims 1-5.

12. A process for the production of pharmaceutical preparations as claimed in claim 11, which comprises bringing at least one compound of the formula I or a tolerable salt thereof as claimed in one or more of claims 1-5 into a suitable administration form using suitable excipients and/or vehicles.

13. *Streptomyces* species HAG 004107, DSM 13381.

## Revendications

1. Composé de formule générale I 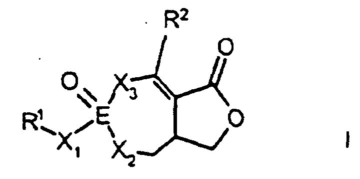 dans laquelle
R¹ représente
1. une chaîne carbonée comprenant 10 à 18 atomes de carbone qui peut être linéaire, ramifiée, saturée ou insaturée, carbocyclique ou hétérocyclique et où la chaîne carbonée est le cas échéant monosubstituée ou disubstituée par
1.1 -OH,
1.2 =O,
1.3 -O-alkyle en C₁ à C₆, où alkyle est linéaire ou ramifié,
1.4 -O-alcényle en C₂ à C₆, où alcényle est linéaire ou ramifié,
1.5 -alkyle en C₁ à C₆, où alkyle est linéaire ou ramifié,
1.6 -aryle,
1.7 -(alkyle en C₁ à C₆)benzène,
1.8 -diphényle,
1.9 -NH-alkyle en C₁ à C₆, où alkyle est linéaire ou ramifié,
1.10 -NH-alcényle en C₂ à C₆, où alcényle est linéaire ou ramifié,
1.11 -NH₂,
1.12 =S,
1.13 -S-alkyle en C₁ à C₆, où alkyle est linéaire ou ramifié, ou
1.14 -S-alcényle en C₂ à C₆, où alcényle est linéaire ou ramifié,
1.15 halogène,
où les substituants 1.1.à 1.15 peuvent également encore être substitués ;
R² représente
1.0 alkyle en C₁ à C₆, où alkyle est non substitué ou monosubstitué ou disubstitué comme décrit aux points 1.1 à 1.15,
2.0 alcényle en C₂ à C₆, où alcényle est non substitué ou monosubstitué ou disubstitué comme décrit aux points 1.1 à 1.15,
3.0 alcynyle en C₂ à C₆, où alcynyle est non substitué ou monosubstitué ou disubstitué comme décrit aux points 1.1 à 1.15,
E représente un phosphore (P) et
X₁, X₂ et X₃ représentent, indépendamment l'un de l'autre, -O-,
dans toutes leur formes stéréochimiques et les mélanges de ces formes dans tous les rapports, ainsi que ses sels physiologiquement acceptables.

2. Composé de formule I ou un sel physiologiquement acceptable de celui-ci selon la revendication 1, **caractérisé en ce que** R¹ représente
1.0 -(CH₂)₁₅CH₃,
2.0 -(CH₂)₁₃CH(CH₃)₂,
3.0 -(CH₂)₁₁CH(OH)(CH₂)₃CH₃,
4.0 -(CH₂)₁₁CH(OH)CH₂CH(CH₃)₂,
5.0 -(CH₂)₁₂CH(OH)(CH₂)₂CH₃,
6.0 -(CH₂)₁₃CH(OH)CH₂CH₃,
7.0 -(CH₂)₁₄CH(OH)CH₃,
8.0 -(CH₂)₁₅CH₂(OH),
9.0 -(CH₂)₁₆CH₃,
10.0 (CH₂)₁₃C=OCH₂CH₃
11.0 -(CH₂)₁₂C=OCH₂CH₂CH₃
12.0 -(CH₂)₁₁C=OCH₂CH₂CH₂CH₃
13.0 -(CH₂)₁₃CH₃
14.0 -(CH₂)₁₁CH(CH₃)₂
15.0 -(CH₂)₁₄CH₃ ou
16.0 -(CH₂)₁₂CH(CH₃)₂.

3. Composé de formule I ou un sel physiologiquement acceptable de celui-ci selon la revendication 1 ou 2, **caractérisé en ce que** R² représente alkyle en C₁ à C₆.

4. Composé de formule I ou un sel physiologiquement acceptable de celui-ci selon la revendication 3, **caractérisé en ce que** R² représente -CH₃, -CH₂CH₃ ou -CH₂CH₂CH₃.

5. Composé de formule I ou un sel physiologiquement acceptable de celui-ci selon l'une ou plusieurs des revendications 1 à 4, pouvant être préparé en ce qu'on fermente le microorganisme Streptomyces type HAG 004107, DSM 13381, ou une de ses variantes ou un de ses mutants dans des conditions appropriées dans un milieu de culture jusqu'à accumulation d'un ou de plusieurs composés de formule générale I dans le milieu de culture, puis on le ou les isole du milieu de culture et on le ou les transforme le cas échéant en équivalents chimiques et sels physiologiquement acceptables.

6. Procédé pour la préparation d'un composé de formule I ou d'un sel physiologiquement acceptable de celui-ci selon l'une ou plusieurs des revendications 1 à 5, **caractérisé en ce que** le microorganisme Streptomyces type HAG 004107, DSM 13381, ou une de ses variantes ou un de ses mutants est fermenté dans des conditions appropriées dans un milieu de culture jusqu'à accumulation d'un ou de plusieurs composés de formule générale I dans le milieu de culture, puis on le ou les isole du milieu de culture et on le ou les transforme le cas échéant en équivalents chimiques et sels physiologiquement acceptables.

7. Procédé selon la revendication 6, **caractérisé en ce que** la fermentation est réalisée dans des conditions aérobies à une température entre 18 et 35°C et à un pH entre 6 et 8.

8. Composé de formule I ou un sel physiologiquement acceptable de celui-ci selon l'une ou plusieurs des revendications 1 à 5 destiné à une utilisation comme médicament.

9. Composé de formule I ou un sel physiologiquement acceptable de celui-ci selon l'une ou plusieurs des revendications 1 à 5 destiné à une utilisation comme médicament pour l'inhibition des lipases.

10. Composé de formule I ou un sel physiologiquement acceptable de celui-ci selon l'une ou plusieurs des revendications 1 à 5 destiné à une utilisation comme antidiabétique.

11. Préparation pharmaceutique présentant une teneur en au moins un composé de formule I ou un sel physiologiquement acceptable de celui-ci selon l'une ou plusieurs des revendications 1 à 5.

12. Procédé pour la préparation de préparations pharmaceutiques selon la revendication 11, **caractérisé en ce qu'**on amène au moins un composé de formule I ou un sel acceptable de celui-ci selon l'une ou plusieurs des revendications 1 à 5 avec des adjuvants et/ou des substances support appropriés dans une forme d'administration appropriée.

13. Streptomyces type HAG 004107, DSM 13381.
